(19) Europäisches Patentamt
European Patent Office
Office européen des brevets

(11) Veröffentlichungsnummer: **0 180 754**
**A2**

(12) # EUROPÄISCHE PATENTANMELDUNG

(21) Anmeldenummer: **85112139.2**

(22) Anmeldetag: **25.09.85**

(51) Int. Cl.[4]: **C 07 D 271/10**
C 07 D 285/12, A 61 K 31/41
C 07 D 417/12
//C07D295/08, C07D211/14,
C07D207/06

(30) Priorität: **09.11.84 DE 3441086**

(43) Veröffentlichungstag der Anmeldung:
**14.05.86 Patentblatt 86/20**

(84) Benannte Vertragsstaaten:
**AT BE CH DE FR GB IT LI LU NL SE**

(71) Anmelder: **LUDWIG HEUMANN & CO GMBH**
**Heideloffstrasse 18-28 Postfach 2260**
**D-8500 Nürnberg(DE)**

(72) Erfinder: **Wegner, Kurt, Dr.**
**Gänsmarkt 3**
**D-6500 Mainz 22(DE)**

(72) Erfinder: **Krämer, Irene**
**Walpodenstrasse 14**
**D-6500 Mainz 1(DE)**

(72) Erfinder: **Schickaneder, Helmut, Dr. Dipl.-Chem.**
**Moosäcker 25**
**D-8501 Eckental(DE)**

(72) Erfinder: **Schunack, Helmut, Prof. Dr.**
**Spanische Allee 95**
**D-1000 Berlin 38(DE)**

(72) Erfinder: **Szelenyi, Istvan, Dr.**
**Händelstrasse 32**
**D-8501 Schwaig(DE)**

(72) Erfinder: **Ahrens, Kurt Henning, Dr.**
**Praterstrasse 9**
**D-8500 Nürnberg(DE)**

(74) Vertreter: **Kraus, Walter, Dr. et al,**
**Patentanwälte Kraus, Weisert & Partner**
**Thomas-Wimmer-Ring 15**
**D-8000 München 22(DE)**

(54) **3,4-Diazolderivate, Verfahren zu ihrer Herstellung und diese Verbindungen enthaltende Arzneimittel.**

(57) Es werden 3,4-Diazolderivate der allgemeinen Formel I

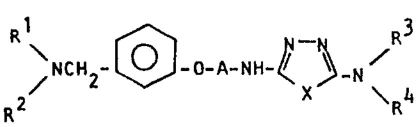

(I)

beschrieben, welche eine hohe selektive Wirkung auf Histamin-$H_2$-Rezeptoren ausüben. Weiterhin werden Verfahren zur Herstellung dieser Verbindungen und diese Verbindungen enthaltende Arzneimittel beschrieben.

EP 0 180 754 A2

# 3,4-Diazolderivate, Verfahren zu ihrer Herstellung und diese Verbindungen enthaltende Arzneimittel

## BESCHREIBUNG

Die Erfindung betrifft neue 3,4-Diazolderivate mit einer hohen selektiven Wirkung auf Histamin-$H_2$-Rezeptoren, Verfahren zu ihrer Herstellung und Arzneimittel, die diese Verbindungen enthalten, sowie schließlich die Verwendung dieser Verbindungen in der Therapie.

Als Anti-Ulcusmittel haben Cimetidin und Ranitidin bereits Eingang in die Therapie gefunden. Die Halbwertszeiten von Cimetidin und Ranitidin sind jedoch verhältnismäßig kurz. Dadurch wird es nötig, mehrfach täglich Tabletten mit Dosiseinheiten von 160 - 300 mg in einer therapeutisch festgelegten Form zu verabreichen. Es besteht somit ein Bedarf an Anti-Ulcusmitteln, die länger wirken und/oder wirksamer sind als Cimetidin und Ranitidin. Die erfindungsgemäßen Verbindungen zeigen aufgrund ihrer spezifischen $H_2$-antagonistischen Aktivität eine Hemmung der Magensäuresekretion, wenn diese durch Histaminagonisten stimuliert wird.

Ash und Shild, "Brit. J. Pharmacol. Chemother.", 27, 427 (1966) und Black et al., "Nature", 236, 385 (1972).

Die pharmakologische Aktivität dieser Verbindungen kann nach einer modifizierten Methode gemäß der DE OS 2734070 beim perfundierten Rattenmagen gezeigt werden oder durch die Ermittlung der $pA_2$-Werte in vitro am Meerschweinchenvorhof (vergleiche Ariens, Molec. Pharmac., Band 1, Academic Press, New York, 1964) nachgewiesen werden.

Weiterhin kann die $H_2$-antagonistische Wirkung an wachen Heidenhain-Pouch-Hunden nach der Methode von Black et al., "Nature" 236, 385 (1972) und an wachen Fistelkatzen gezeigt werden.

Die neuen Verbindungen antagonisieren weiterhin die Histaminwirkung auf die Kontraktionsfrequenz des isolierten rechten Atriums des Meerschweinchens, aber sie beeinflussen nicht histamininduzierte Kontraktionen des isolierten, glatten gastrointestinalen Muskels, wenn diese durch $H_2$-Agonisten hervorgerufen werden. Nachdem Hemmstoffe für Histamin-$H_2$-Rezeptoren sowohl bezüglich der basalen Magensäuresekretion als auch der durch Gastrin, Histamin, Metacholin oder Nahrung induzierten Magensäuresekretion Hemmwirkung besitzen, können sie für die Behandlung von peptischen Ulcera, die durch übermäßige Sekretion von Magensäure verursacht sind, und bei der Therapie hyperacider Gastritis verwendet werden.

Der Erfindung liegt die Aufgabe zugrunde, neue Hemmstoffe für Histamin-$H_2$-Rezeptoren mit verbesserter und/oder länger anhaltender Wirksamkeit zur Verfügung zu stellen. Diese Aufgabe wird durch die Erfindung gelöst.

Gegenstand der Erfindung sind daher neue 3,4-Diazolderivate der allgemeinen Formel I

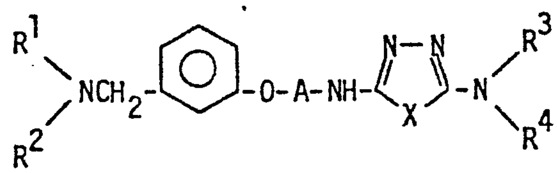

(I)

in der $R^1$ und $R^2$, die gleich oder verschieden sein können, jeweils für Wasserstoff, lineares $C_1$-$C_{10}$-Alkyl, $C_5$-$C_6$-Cycloalkyl, Amino, $C_1$-$C_3$-Alkylamino oder Di($C_1$-$C_3$-alkyl)amino stehen oder $R^1$ und $R^2$ zusammen mit dem Stickstoffatom, an das sie gebunden sind, einen gegebenenfalls ein- oder zweifach mit einer $C_1$-$C_3$-Alkylgruppe substituierten 5- bis 10-gliedrigen stickstoffhaltigen alicyclischen, heterocyclischen Ring bilden, A die Gruppierung $-(CH_2)_n-$, $-CH_2-CH(CH_3)-CH_2-$, $-CH_2-CH{=}CH-CH_2-$ oder $-CH_2-CH(OH)-CH_2-$ bedeutet, wobei n für 3 oder 4 steht, X ein Sauerstoff- oder Schwefelatom bedeutet, $R^3$ und $R^4$, die gleich oder verschieden sein können, jeweils Wasserstoff, lineares oder verzweigtkettiges $C_1$-$C_6$-Alkyl, $C_5$-$C_6$-Cycloalkyl oder eine Benzyl-, Benzoyl- oder Phenylgruppe, die gegebenenfalls durch eine Nitro-, Halogen- oder $C_1$-$C_3$-Alkylgruppe substituiert ist, bedeuten oder $R^3$ und $R^4$ zusammen mit dem Stickstoffatom, an das sie gebunden sind, einen 5- bis 7gliedrigen stickstoffhaltigen alicyclischen, heterocyclischen Ring bilden, sowie ihre physiologisch annehmbaren Salze.

In der allgemeinen Formel I bedeuten die Substituenten $R^1$ und $R^2$, die gleich oder verschieden sein können, jeweils ein Wasserstoffatom, eine lineare $C_1$-$C_{10}$-Alkylgruppe, vorzugsweise eine lineare $C_1$-$C_6$-Alkylgruppe, ganz besonders bevorzugt eine lineare $C_1$-$C_3$-Alkylgruppe, eine $C_5$-$C_6$-Cycloalkylgruppe, d.h. eine Cyclopentyl- oder Cyclohexylgruppe, eine Aminogruppe, eine $C_1$-$C_3$-Alkylaminogruppe, zum Beispiel eine n- oder i-Propylaminogruppe, eine Ethylaminogruppe oder eine Methylaminogruppe, wobei die Methylaminogruppe bevorzugt wird, oder eine Di-

($C_1$-$C_3$-alkyl)aminogruppe, vorzugsweise eine Diethyl- oder Dimethylaminogruppe. $R^1$ und $R^2$ können aber auch zusammen mit dem Stickstoffatom, an das sie gebunden sind, einen gegebenenfalls ein- oder zweifach mit einer $C_1$-$C_3$-Alkylgruppe substituierten 5- bis 10gliedrigen stickstoffhaltigen alicyclischen, heterocyclischen Ring bilden. Dieser Ring kann gegebenenfalls ein oder mehrere weitere Heteroatome, zum Beispiel ein weiteres Stickstoffatom, ein Sauerstoffatom oder ein Schwefelatom, enthalten. Bevorzugte Beispiele für den so definierten 5- bis 10gliedrigen heterocyclischen Ring sind der Pyrrolidin-, Piperidin- und Homopiperidinring. Bevorzugte Beispiele für heterocyclische Ringe, die weitere Heteroatome enthalten, sind der Piperazin- und der Morpholinring. Im Falle einer Ein- oder Zweifachsubstitution am 5- bis 10gliedrigen stickstoffhaltigen alicyclischen, heterocyclischen Ring bedeuten die Substituenten jeweils eine $C_1$-$C_3$-Alkylgruppe, vorzugsweise eine Methylgruppe. So kann zum Beispiel der Pyrrolidin-, Piperidin- oder Homopiperidinring jeweils in 2-, 3- oder 4-Stellung oder bei einer Zweifachsubstitution in 2,5- oder 2,6-Stellung mit einer Methylgruppe substituiert sein. Bevorzugte substituierte stickstoffhaltige 5- bis 10gliedrige alicyclische, heterocyclische Ringe sind zum Beispiel der 3-Methylpyrrolidin-, 3-Methylpiperidin-, 4-Methylpiperidin- oder 3,5-Dimethylpiperidinring, ganz besonders bevorzugt sind der 3-Methylpyrrolidin- und 3-Methylpiperidinring.

A bedeutet eine der folgenden Gruppierungen:

$-(CH_2)_n-$, $-CH_2-CH(CH_3)-CH_2-$, $-CH_2-CH{=}CH-CH_2-$ oder $-CH_2-CH(OH)-CH_2-$. n bedeutet eine ganze Zahl 3 oder 4, wobei der Wert 3 bevorzugt wird. X ist ein Sauerstoff- oder Schwefelatom, wobei das Schwefelatom bevorzugt wird. Die Substituenten $R^3$ und $R^4$ können ebenfalls gleich oder verschieden sein. $R^3$ und $R^4$ bedeuten jeweils Wasserstoff, eine lineare oder verzweigtkettige $C_1$-$C_6$-Alkylgruppe, vorzugsweise eine $C_1$-$C_3$-Alkylgruppe, wie zum Beispiel eine i- oder n-Propylgruppe, eine Ethyl- oder Methylgruppe, insbesondere eine Methylgruppe, eine $C_5$-$C_6$-Cycloalkylgruppe, d.h. eine Cyclopentyl- oder Cyclohexylgruppe, oder eine Benzyl-, Benzoyl- oder Phenylgruppe. Die Benzyl-, Benzoyl- oder Phenylgruppe kann unsubstituiert sein oder gegebenenfalls durch eine Nitrogruppe, ein Ha-

logenatom oder eine $C_1$-$C_3$-Alkylgruppe, insbesondere eine Methylgruppe, substituiert sein. $R^3$ und $R^4$ können auch zusammen mit dem Stickstoffatom, an das sie gebunden sind, einen 5- bis 7gliedrigen stickstoffhaltigen alicyclischen, heterocyclischen Ring bilden, wobei der Piperidinring bevorzugt wird. Der so gebildete Ring kann ein weiteres Heteroatom, beispielsweise ein Stickstoffatom, ein Sauerstoff- oder Schwefelatom, enthalten. Ein bevorzugtes Beispiel für einen derartigen Ring ist der Morpholin- oder der Piperazinring.

Eine bevorzugte Gruppe von erfindungsgemäßen Verbindungen ist dadurch gekennzeichnet, daß einer der Substituenten $R^1$ und $R^2$ ein Wasserstoffatom und der andere eine $C_1$-$C_3$-Alkylgruppe, vorzugsweise eine Methyl-

gruppe, ist, wobei A, X, $R^3$ und $R^4$ wie oben definiert sind. Eine weitere bevorzugte Gruppe von erfindungsgemäßen Verbindungen ist dadurch gekennzeichnet, daß $R^1$ und $R^2$ jeweils für eine $C_1$-$C_3$-Alkylgruppe, insbesondere eine Methylgruppe, stehen, wobei A, X, $R^3$ und $R^4$ wie oben definiert sind.

Die Erfindung umfaßt auch alle stereoisomeren Formen und Hydrate der oben beschriebenen Verbindungen der allgemeinen Formel I.

Die erfindungsgemäßen Verbindungen können durch ein Verfahren hergestellt werden, das dadurch gekennzeichnet ist, daß man

(a) zur Herstellung von Verbindungen, bei denen $R^1$, $R^2$ und A die oben angegebenen Bedeutungen haben, $R^3$ für Wasserstoff steht, $R^4$ lineares oder verzweigtkettiges $C_1$-$C_6$-Alkyl, $C_5$-$C_6$-Cycloalkyl oder eine Benzyl-, Benzoyl- oder Phenylgruppe, die gegebenenfalls durch eine Nitro-, Halogen- oder $C_1$-$C_3$-Alkylgruppe substituiert ist, bedeutet und X für ein Sauerstoff- oder Schwefelatom steht, eine Verbindung der allgemeinen Formel II

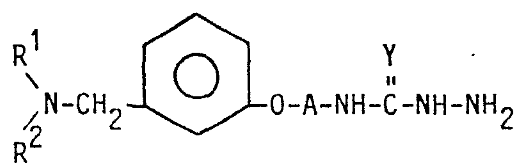

(II)

in der $R^1$, $R^2$ und A wie oben definiert sind und Y für ein Sauerstoff- oder Schwefelatom steht, mit einem Isocyaniddichlorid der allgemeinen Formel III

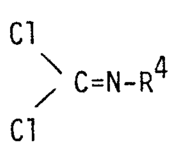

(III)

in der $R^4$ wie oben definiert ist, zu einer Verbindung der allgemeinen Formel I umsetzt oder daß man

(b) zur Herstellung von Verbindungen, bei denen $R^1$, $R^2$ und A die oben angegebenen Bedeutungen haben, $R^3$ und $R^4$, die gleich oder verschieden sein können, jeweils lineares oder verzweigtkettiges $C_1$-$C_6$-Alkyl, $C_5$-$C_6$-Cycloalkyl oder eine Benzyl-, Benzoyl- oder Phenylgruppe, die gegebenenfalls durch eine Nitro-, Halogen- oder $C_1$-$C_3$-Alkylgruppe substituiert ist, bedeuten oder $R^3$ und $R^4$ zusammen mit dem Stickstoffatom, an das sie gebunden sind, einen 5- bis 7gliedrigen stickstoffhaltigen alicyclischen, heterocyclischen Ring bilden und X für ein Schwefelatom steht, eine Verbindung der allgemeinen Formel IV

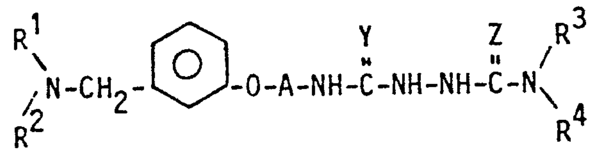

(IV)

in der $R^1$, $R^2$, $R^3$, $R^4$ und A wie oben definiert sind und Y und Z jeweils für ein Schwefelatom stehen, mit Hilfe eines Oxidationsmittels zu einer Verbindung der allgemeinen Formel I cyclisiert oder daß man

(c) zur Herstellung von Verbindungen, bei denen $R^1$, $R^2$ und A die oben angegebenen Bedeutungen haben, $R^3$ und $R^4$, die gleich oder verschieden sein können, jeweils lineares oder verzweigtkettiges $C_1$-$C_6$-Alkyl, $C_5$-$C_6$-Cycloalkyl oder eine Benzyl-, Benzoyl- oder Phenylgruppe, die gegebenenfalls durch eine Nitro-, Halogen- oder $C_1$-$C_3$-Alkylgruppe substituiert ist, bedeuten oder $R^3$ und $R^4$ zusammen mit dem Stickstoffatom, an das sie gebunden sind, einen 5- bis 7gliedrigen stickstoffhaltigen alicyclischen, heterocyclischen Ring bilden und X für ein Schwefelatom steht, eine Verbindung der allgemeinen Formel IV

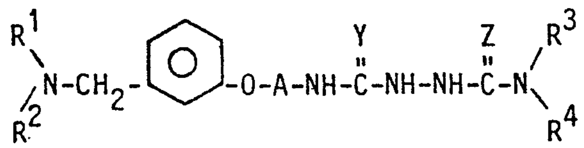

(IV)

in der $R^1$, $R^2$, $R^3$, $R^4$ und A wie oben definiert sind und entweder Y und Z gleich sind und jeweils für ein Schwefelatom stehen oder Y und Z voneinander verschieden sind und entweder für ein Sauerstoff- oder ein Schwefelatom stehen, mit Phosphoroxychlorid zu einer Verbindung der allgemeinen Formel I cyclisiert oder daß man

(d) zur Herstellung von Verbindungen, bei denen $R^1$, $R^2$ und A die oben angegebenen Bedeutungen haben, $R^3$ und $R^4$, die gleich oder verschieden sein können, jeweils lineares oder verzweigtkettiges $C_1$-$C_6$-Alkyl, $C_5$-$C_6$-Cycloalkyl oder eine Benzyl-, Benzoyl- oder Phenylgruppe, die gegebenenfalls durch eine Nitro-, Halogen- oder $C_1$-$C_3$-Alkylgruppe substituiert ist, bedeuten oder $R^3$ und $R^4$ zusammen mit dem Stickstoffatom, an das sie gebunden sind, einen 5- bis 7gliedrigen stickstoffhaltigen, alicyclischen, heterocyclischen Ring bilden und X für ein Sauerstoffatom steht, eine Verbindung der allgemeinen Formel IV

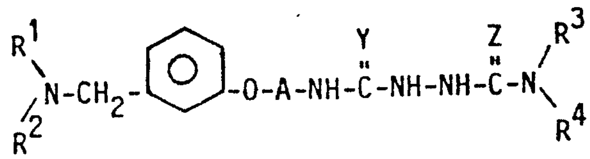

(IV)

in der $R^1$, $R^2$, $R^3$, $R^4$ und A wie oben definiert sind und Y und Z jeweils für ein Sauerstoffatom stehen, mit Phosphoroxychlorid zu einer Verbindung der allgemeinen Formel I cyclodehydratisiert oder daß man

(e) zur Herstellung von Verbindungen, bei denen $R^1$, $R^2$ und A die oben angegebenen Bedeutungen haben, $R^3$ und $R^4$ jeweils Wasserstoff bedeuten und X für ein Sauerstoff- oder Schwefelatom steht, eine Verbindung der allgemeinen Formel V

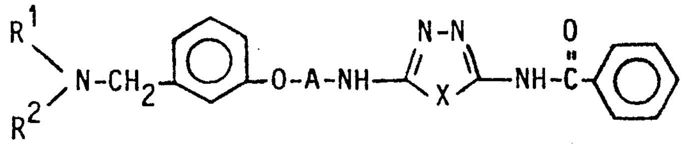

(V)

in der $R^1$, $R^2$ und A wie oben definiert sind, durch Hydrolyse zu einer Verbindung der allgemeinen Formel I umsetzt
oder daß man

(f) zur Herstellung von Verbindungen, bei denen $R^1$, $R^2$, $R^3$, $R^4$ und A die oben angegebenen Bedeutungen haben und X für ein Schwefelatom steht, ein Amin der allgemeinen Formel VI

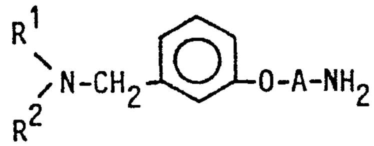
(VI)

in der $R^1$, $R^2$ und A wie oben definiert sind, mit einem 1,3,4-Thiadiazol-2-amin der allgemeinen Formel VII

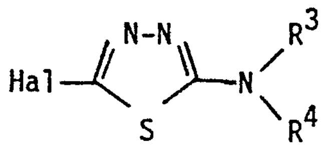
(VII)

in der Hal für ein Halogenatom steht und $R^3$ und $R^4$ wie oben definiert sind, zu einer Verbindung der allgemeinen Formel I umsetzt
oder daß man

(g) zur Herstellung von Verbindungen, bei denen $R^1$, $R^2$, $R^3$, $R^4$ und A die oben angegebenen Bedeutungen haben und X für ein Schwefelatom steht, eine Verbindung der allgemeinen Formel VIII

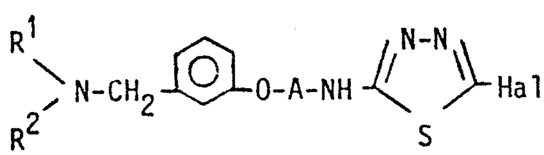
(VIII)

in der $R^1$, $R^2$ und A wie oben definiert sind und Hal für ein Halogenatom steht, mit einem Amin der allgemeinen Formel IX

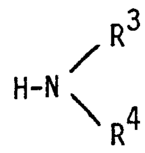

(IX)

in der $R^3$ und $R^4$ wie oben definiert sind, zu einer Verbindung der allgemeinen Formel I umsetzt

und daß man die in den Stufen (a) bis (g) erhaltene Verbindung gegebenenfalls in ihr physiologisch unbedenkliches Salz umwandelt.

Die Synthese gemäß der Variante (a) erfolgt in an sich bekannter Weise. Vorteilhafterweise wird die Umsetzung in einem Lösungsmittel, bevorzugt in einem aprotischen Lösungsmittel, zum Beispiel Dioxan oder Tetrahydrofuran, durchgeführt. Die Umsetzung erfolgt vorzugsweise bei erhöhter Temperatur, beispielsweise bei Rückflußtemperatur des verwendeten Lösungsmittels, und nimmt im allgemeinen einen Zeitraum von 1 bis 3 Stunden in Anspruch. Die Isolierung und Aufarbeitung sowie die Reinigung erfolgen ebenfalls in an sich bekannter Weise, beispielsweise durch präparative Schichtchromatographie.

Die Synthese gemäß der Variante (b) erfolgt ebenfalls in an sich bekannter Weise. Auch hier wird vorzugsweise ein Lösungsmittel, beispielsweise ein aprotisches Lösungsmittel, wie oben definiert, verwendet. Als Oxidationsmittel wird vorzugsweise Hydroperoxid eingesetzt. Die Aufarbeitung und Isolierung erfolgt in an sich bekannter Weise, beispielsweise durch präparative Dickschichtchromatographie.

Das gleiche gilt für die Varianten (c) bis (g), die ebenfalls in an sich bekannter Weise durchgeführt werden.

Die Herstellung der unter (a) bis (g) beschriebenen Ausgangsprodukte kann prinzipiell nach bekannten Synthesevorschriften für 1,3,4-Oxa-(thia)diazol-2,5-diamine durchgeführt werden (siehe

1. W.R. Sherman in "Heterocyclic Compounds", Bd. 7, 541 - 626 (Hrsg.: R.C. Elderfield, Wiley, New York, 1961)

2. J. Sandström in "Advances in Heterocyclic Chemistry", Bd. 9, 165 - 209 (Hrsg.: A.R. Katritzky und A.J. Boulton, Academic Press, New York (1968), Recent advances in the chemistry of 1,3,4-thiadiazoles

3. A. Hetzheim und K. Möckel in "Advances in Heterocyclic Chemistry", Bd. 7 (Hrsg.: A.R. Katritzky und A.J. Boulton, Academic Press, New York, 1968)

4. G. Werber u.a., J. Heterocycl. Chem. 14, 823 (1977)).

Es hat sich gezeigt, daß diese Ausgangsprodukte ebenfalls eine gewisse $H_2$-antagonistische Aktivität zeigen, so daß gegebenenfalls deren therapeutische Verwendbarkeit in Betracht gezogen werden könnte.

Die Erfindung umfaßt neben den stereoisomeren Verbindungen und Hydraten der Substanzen der allgemeinen Formel I auch die physiologisch annehmbaren Salze dieser Verbindungen. Diese Salze können zum Beispiel mit Mineralsäuren, wie Chlor-, Brom-, Jodwasserstoffsäure, Phosphorsäure, Metaphosphorsäure, Salpetersäure oder Schwefelsäure, oder mit organischen Säuren, wie Ameisensäure, Essigsäure, Propionsäure, Phenylessigsäure, Weinsäure, Zitronensäure, Fumarsäure, Methansulfonsäure etc., gebildet werden.

Wie oben bereits ausgeführt, stellen die erfindungsgemäßen Verbindungen wirksame $H_2$-Antagonisten dar, die anerkannt guten Verbindungen gleicher Wirkungsrichtung, wie beispielsweise dem CIMETIDIN und dem RANITIDIN, überlegen sind.

Ein weiterer Gegenstand der Erfindung ist daher ein Arzneimittel, insbesondere zur Behandlung von gastrointestinalen Erkrankungen, das dadurch gekennzeichnet ist, daß es als Wirkstoff mindestens eine der oben beschriebenen Verbindungen zusammen mit einem inerten pharmazeutisch annehmbaren Träger oder Verdünnungsmittel enthält.

Die erfindungsgemäßen Verbindungen können zur Verabreichung in jeder beliebigen Weise formuliert werden. Die Erfindung umfaßt daher auch Arzneimittel, die mindestens eine erfindungsgemäße Verbindung zur Verwendung in der Human- oder Veterinärmedizin enthalten. Solche Arzneimittel können herkömmlicherweise unter Verwendung von einem oder mehreren pharmazeutischen Trägern oder Verdünnungsmitteln hergestellt werden.

Die erfindungsgemäßen Verbindungen können daher für die orale, bukkale, topische, parenterale oder rektale Verabreichung formuliert werden, wobei die orale Verabreichung bevorzugt wird.

Für die orale Verabreichung kann das Arzneimittel in Form von beispielsweise Tabletten, Kapseln, Pulvern, Lösungen, Sirups oder Suspensionen vorliegen, die unter Verwendung von annehmbaren Verdünnungsmitteln auf herkömmliche Weise hergestellt worden sind.

Für die bukkale Verabreichung kann das Arzneimittel die Form von Tabletten oder Briefchen einnehmen, die in herkömmlicher Weise formuliert worden sind.

Die erfindungsgemäßen Verbindungen können für die parenterale Verabreichung durch Bolusinjektion oder kontinuierliche Infusion formuliert werden. Formulierungen zur Injektion können in Dosiseinheitsform als Ampullen oder in Mehrfachdosenbehältern mit zugesetztem Konservierungsmittel vorliegen.

Die Arzneimittel können solche Formen, wie Suspensionen, Lösungen oder Emulsionen in öligen oder wäßrigen Trägern, einnehmen, und sie können Formulierungshilfsmittel, wie Suspendierungs-, Stabilisierungs- und/ oder Dispergierungsmittel, enthalten.

Alternativ kann der Wirkstoff auch in Pulverform zur Rekonstitution mit einem geeigneten Träger, zum Beispiel sterilem, pyrogenfreiem Wasser, vor dem Gebrauch vorliegen.

Die erfindungsgemäßen Verbindungen können auch für rektale Zubereitungen, zum Beispiel Suppositorien oder Retentionseinläufe, formuliert werden, die zum Beispiel herkömmliche Suppositoriengrundlagen, wie Kakaobutter oder andere Glyceride, enthalten.

Zur topischen Anwendung können die erfindungsgemäßen Verbindungen als Salben, Cremes, Gels, Lotionen, Pulver oder Sprays in herkömmlicher Weise formuliert werden.

Für die orale Verabreichung ist eine geeignete Tagesdosis an erfindungsgemäßen Verbindungen 1 bis 4 Dosen bis insgesamt 5 mg bis 1 g/Tag, vorzugsweise 5 bis 250 mg/Tag, je nach Zustand des Patienten. Im Einzelfall kann es gegebenenfalls erforderlich sein, von den genannten Mengen abzuweichen, und zwar in Abhängigkeit vom individuellen Verhalten gegenüber dem Wirkstoff bzw. der Art seiner Formulierung und dem Zeitpunkt bzw. Intervall, zu welchem die Verabreichung erfolgt. So gibt es zum Beispiel Fälle, wo mit weniger als der oben genannten Mindestmenge ausgekommen werden kann, während in anderen Fällen die genannte obere Grenze überschritten werden muß.

Die Erfindung wird in den Beispielen erläutert. Die Beispiele 1 bis 4 beschreiben die Herstellung von Ausgangsverbindungen der allgemeinen Formel II. Die Beispiele 5 bis 28 beschreiben die Herstellung von Ausgangsverbindungen der allgemeinen Formel IV. Die Beispiele 29 bis 70 beschreiben die Herstellung der erfindungsgemäßen Verbindungen der allgemeinen Formel I.

## B e i s p i e l   1

N-[3-[3-(1-Piperidinylmethyl)phenoxy]propyl]hydrazincarboxamid

Zu einer Lösung von 10,0 g (40 mmol) 3-[3-(1-Piperidinylmethyl)phenoxy]-1-propanamin in 200 ml absolutem Ether werden bei Raumtemperatur langsam 6,3 g (40 mmol) Chlorameisensäurephenylester getropft. Gleichzeitig mit der zweiten Hälfte des Esters wird eine Lösung von 1,6 g (40 mmol) Natriumhydroxid in 16 ml Wasser zugetropft. Es wird eine Stunde gerührt, anschließend werden die Phasen getrennt. Die Etherphase wird gewaschen, über Natriumsulfat getrocknet und eingeengt. Der ölige Rückstand wird in 250 ml einer Ethanol/Ether-Mischung (1:1) aufgenommen und bei 0°C bis zur vollständigen Fällung des Produkts mit trockenem Chlorwasserstoff begast.

Ausbeute: 13 g N-[3-[3-(1-Piperidinylmethyl)phenoxy]propyl]carbamidsäurephenylesterhydrochlorid; Schmp.: 144°C

$C_{22}H_{28}N_2O_3 \cdot HCl$ (404,9)

| | | | |
|---|---|---|---|
| Berechnet: | C 65,3 | H 7,22 | N 6,92 |
| Gefunden: | C 65,2 | H 6,97 | N 7,09 |

13 g (32 mmol) des so hergestellten Esters werden unter Zugabe von 4,5 ml (32 mmol) Triethylamin in 100 ml Ethanol gelöst, mit 15 g (0,3 mol) Hydrazinhydrat versetzt und 3 Stunden unter Rückfluß erhitzt. Der Ansatz wird im Vakuum eingeengt, überschüssiges Hydrazinhydrat weitgehend abdestilliert. Der ölige Rückstand wird durch Säulenchromatographie gereinigt (Kieselgel; ammoniakgesättigtes Methylenchlorid).

Ausbeute: 7 g farbloses Öl (72% d.Th.)

$C_{16}H_{26}N_4O_2$ (306,4)

1H-NMR ($CDCl_3$; TMS): $\delta$ = 1,3-1,7 (m) ($-(CH_2)_3-$) 6H; 1,97 (m) ($CH_2$) 2H; 2,2-2,5 (m) ($-(CH_2)_2-$) 4H; 3,1-3,7 (m) (N-$CH_2$, $NH_2$ (austauschbar mit $D_2O$)) 4H; 3,37 (s) ($CH_2$) 2H; 4,00 (t) (O-$CH_2$) 2H; 6,34 (t) (NH (austauschbar mit $D_2O$)) 1H; 6,6-7,3 (m) (Aromaten-H, NH (austauschbar mit $D_2O$)) 5H.

## Beispiel 2

N-[2-Methyl-3-[3-(1-piperidinylmethyl)phenoxy]propyl]hydrazincarboxamid

2,2 g (8 mmol) 2-Methyl-3-[3-(1-piperidinylmethyl)phenoxy]-1-propanamin in 20 ml Ethanol werden mit 1,9 g (8 mmol) Diphenylcarbonat 12 Stunden bei Raumtemperatur gerührt. Anschließend wird die Lösung mit 4 g Hydrazinhydrat versetzt und eine Stunde unter Rückfluß erhitzt. Die Isolierung erfolgt wie in Beispiel 1 beschrieben.

Ausbeute: 1,1 g farbloses Öl (43% d.Th.)

$C_{17}H_{27}N_4O_2$ (319,4)

1H-NMR ($CDCl_3$; TMS): $\delta$ = 1,33 (d) ($CH_3$) 3H; 1,3-1,7 (m) ($-(CH_2)_3-$) 6H; 1,9-2,5 (m) ($-(CH_2)_2-$, CH) 5H; 3,1-3,7 (m) (N-$CH_2$, $NH_2$ (austauschbar

mit $D_2O$)) 4H; 3,40 (s) ($CH_2$) 2H; 3,86 (d) (O-$CH_2$) 2H; 6,40 (t) (NH (austauschbar mit $D_2O$)) 1H; 6,6-7,3 (m) (Aromaten-H, NH (austauschbar mit $D_2O$)) 5H.

B e i s p i e l 3

N-[3-[3-(1-Piperidinylmethyl)phenoxy]propyl]hydrazincarbothioamid

49,7 g (0,2 mol) 3-[3-(1-Piperidinylmethyl)phenoxy]-1-propanamin in 200 ml Ethanol werden unter heftigem Rühren in eine Mischung aus je 200 ml Ethanol und Schwefelkohlenstoff getropft und über Nacht weitergerührt. Nach Verdampfen des Schwefelkohlenstoffs wird die ethanolische Suspension mit 30 g (0,21 mol) Methyljodid versetzt und bis zur vollständigen Auflösung gerührt. Man engt dann auf ca. 200 ml ein, gibt 50 ml Hydrazinhydrat hinzu und läßt bei Raumtemperatur 48 Stunden stehen. Das entstandene Kristallisat wird abgesaugt und mit kaltem Ethanol gewaschen.

Ausbeute: 51 g (79% d.Th.)

$C_{16}H_{26}N_4OS$ (322,5); Schmp.: 116°C (Ethanol)

| | | | |
|---|---|---|---|
| Berechnet: | C 59,6 | H 8,13 | N 17,4 |
| Gefunden: | C 59,4 | H 8,04 | N 17,3 |

B e i s p i e l 4

N-[2-Hydroxy-3-[3-(1-piperidinylmethyl)phenoxy]propyl]hydrazincarbothioamid

3,97 g (15 mmol) 1-Amino-3-[3-(1-piperidinylmethyl)phenoxy]-2-propanol werden analog Beispiel 3 umgesetzt. Nach Eindampfen der Reaktionslösung wird der ölige Rückstand mehrmals mit Wasser digeriert, zentrifugiert, getrocknet und mit Ether zur Kristallisation gebracht.

Ausbeute: 2,5 g (67% d.Th.)

$C_{16}H_{26}N_4O_2S$ (338,5); Schmp.: 91-93°C (Acetonitril/Ether)

| | | | |
|---|---|---|---|
| Berechnet: | C 56,8 | H 7,74 | N 16,6 |
| Gefunden: | C 56,6 | H 7,65 | N 16,6 |

Beispiel 5

N-Methyl-N'-[3-[3-(1-piperidinylmethyl)phenoxy]propyl]-1,2-hydrazindicarbothioamid

1,62 g (5 mmol) N-[3-[3-(1-Piperidinylmethyl)phenoxy]propyl]hydrazincarbothioamid werden in 30 ml THF gelöst, mit 0,44 g (6 mmol) Methylisothiocyanat versetzt und bei Raumtemperatur über Nacht stehen gelassen. Zur Vervollständigung der Fällung wird mit Ether versetzt, der Niederschlag abgesaugt, mit Ether gewaschen und aus Ethanol umkristallisiert. Die Analysenwerte sind in Tabelle I zusammengestellt.

Beispiel 6

N-Ethyl-N'-[3-[3-(1-piperidinylmethyl)phenoxy]propyl]-1,2-hydrazindicarbothioamid

Die Herstellung erfolgt analog Beispiel 5 aus N-[3-[3-(1-Piperidinylmethyl)phenoxy]propyl]hydrazincarbothioamid und Ethylisothiocyanat. Die Analysenwerte sind in Tabelle I zusammengestellt.

Beispiel 7

N-Cyclohexyl-N'-[3-[3-(1-piperidinylmethyl)phenoxy]propyl]-1,2-hydrazindicarbothioamid

Die Herstellung erfolgt analog Beispiel 5 aus N-[3-[3-(1-Piperidinylmethyl)phenoxy]propyl]hydrazincarbothioamid und Cyclohexylisothiocyanat. Die Analysenwerte sind in Tabelle I zusammengestellt.

Beispiel 8

N-Benzoyl-N'-[3-[3-(1-piperidinylmethyl)phenoxy]propyl]-1,2-hydrazindicarbothioamid

Die Herstellung erfolgt analog Beispiel 5 aus N-[3-[3-(1-piperidinylmethyl)phenoxy]propyl]hydrazincarbothioamid und Benzoylisothiocyanat. Die Analysenwerte sind in Tabelle I zusammengestellt.

Beispiel 9

N-Methyl-2-[[3-[3-(1-piperidinylmethyl)phenoxy]propyl]aminothioxomethyl]-hydrazincarboxamid

1,62 g (5 mmol) N-[3-[3-(1-Piperidinylmethyl)phenoxy]propyl]hydrazincarbothioamid werden in 30 ml THF gelöst, mit 0,40 g (6 mmol) Methylisocyanat versetzt und bei Raumtemperatur über Nacht stehen gelassen. Zur Vervollständigung der Fällung wird mit Ether versetzt, der Niederschlag abgesaugt, mit Ether gewaschen und aus Ethanol umkristallisiert. Die Analysenwerte sind in Tabelle I zusammengestellt.

Beispiel 10

N-Ethyl-2-[[3-[3-(1-piperidinylmethyl)phenoxy]propyl]aminothioxomethyl]-hydrazincarboxamid

Die Herstellung erfolgt analog Beispiel 9 aus N-[3-[3-(1-Piperidinylmethyl)phenoxy]propyl]hydrazincarbothioamid und Ethylisocyanat. Die Analysenwerte sind in Tabelle I zusammengestellt.

Beispiel 11

N-Isopropyl-2-[[3-[3-(1-piperidinylmethyl)phenoxy]propyl]aminothioxomethyl]-hydrazincarboxamid

Die Herstellung erfolgt analog Beispiel 9 aus N-[3-[3-(1-Piperidinylmethyl)phenoxy]propyl]hydrazincarbothioamid und Isopropylisocyanat. Die Analysenwerte sind in Tabelle I zusammengestellt.

Beispiel 12

N-Butyl-2-[[3-[3-(1-piperidinylmethyl)phenoxy]propyl]aminothioxomethyl]-hydrazincarboxamid

Die Herstellung erfolgt analog Beispiel 9 aus N-[3-[3-(1-Piperdinylmethyl)phenoxy]propyl]hydrazincarbothioamid und n-Butylisocyanat. Die Analysenwerte sind in Tabelle I zusammengestellt.

Beispiel 13

N-Cyclohexyl-2-[[3-[3-(1-piperidinylmethyl)phenoxy]propyl]aminothioxomethyl]-hydrazincarboxamid

Die Herstellung erfolgt analog Beispiel 9 aus N-[3-[3-(1-Piperidinylmethyl)phenoxy]propyl]hydrazincarbothioamid und Cyclohexylisocyanat. Die Analysenwerte sind in Tabelle I zusammengestellt.

Beispiel 14

N-Benzyl-2-[[3-[3-(1-piperidinylmethyl)phenoxy]propyl]aminothioxomethyl]-hydrazincarboxamid

Die Herstellung erfolgt analog Beispiel 9 aus N-[3-[3-(1-Piperidinylmethyl)phenoxy]propyl]hydrazincarbothioamid und Benzylisocyanat. Die Analsenwerte sind in Tabelle I zusammengestellt.

Beispiel 15

N-Phenyl-2-[[3-[3-(1-piperidinylmethyl)phenoxy]propyl]aminothioxomethyl]-hydrazincarboxamid

Die Herstellung erfolgt analog Beispiel 9 aus N-[3-[3-(1-Piperidinylmethyl)phenoxy]propyl]hydrazincarbothioamid und Phenylisocyanat. Die Analysenwerte sind in Tabelle I zusammengestellt.

Beispiel 16

N-4-Chlorphenyl-2-[[3-[3-(1-piperidinylmethyl)phenoxy]propyl]aminothioxomethyl]hydrazincarboxamid

Die Herstellung erfolgt analog Beispiel 9 aus N-[3-[3-(1-Piperidinylmethyl)phenoxy]propyl]hydrazincarbothioamid und 4-Chlorphenylisocyanat. Die Analysenwerte sind in Tabelle I zusammengestellt.

### Beispiel 17

2-(Methylaminothioxomethyl)-N-[3-[3-(1-piperidinylmethyl)phenoxy]-propyl]hydrazincarboxamid

Zu 1,0 g (3,3 mmol) N-[3-[3-(1-Piperidinylmethyl)phenoxy]propyl]hydrazincarboxamid in 20 ml THF werden 0,5 g (6,6 mmol) Methylisothiocyanat gegeben und 12 Stunden bei Raumtemperatur gerührt. Der Niederschlag wird abfiltriert und aus Ethanol umkristallisiert. Die Analysenwerte sind in Tabelle I zusammengestellt.

### Beispiel 18

2-(Ethylaminothioxomethyl)-N-[3-[3-(1-piperidinylmethyl)phenoxy]propyl]-hydrazincarboxamid

Die Herstellung erfolgt analog Beispiel 17 aus N-[3-[3-(1-Piperidinylmethyl)phenoxy]propyl]hydrazincarboxamid und Ethylisothiocyanat. Die Analysenwerte sind in Tabelle I zusammengestellt.

### Beispiel 19

2-(Cyclohexylaminothioxomethyl)-N-[3-[3-(1-piperidinylmethyl)phenoxy]-propyl]-hydrazincarboxamid

Die Herstellung erfolgt analog Beispiel 17 aus N-[3-[3-(1-Piperidinylmethyl)phenoxy]propyl]hydrazincarboxamid und Cyclohexylisothiocyanat. Die Analysenwerte sind in Tabelle I zusammengestellt.

0180754

B e i s p i e l 20

N-Methyl-N'-[3-[3-(1-piperidinylmethyl)phenoxy]propyl]-1,2-hydrazindicarboxamid

Zu 2,0 g (6,6 mmol) N-[3-[3-(1-Piperidinylmethyl)phenoxy]propyl]hydrazincarboxamid in 20 ml THF werden 0,8 ml (13 mmol) Methylisocyanat gegeben und 1 Stunde bei Raumtemperatur gerührt. Der Niederschlag wird abfiltriert und aus Methanol umkristallisiert. Die Analysenwerte sind in Tabelle I zusammengestellt.

B e i s p i e l 21

N-Ethyl-N'-[3-[3-(1-piperidinylmethyl)phenoxy]propyl]-1,2-hydrazindicarboxamid

Die Herstellung erfolgt analog Beispiel 20 aus N-[3-[3-(1-Piperidinylmethyl)phenoxy]propyl]hydrazincarboxamid und Ethylisocyanat. Die Analysenwerte sind in Tabelle I zusammengestellt.

B e i s p i e l 22

N-Isopropyl-N'-[3-[3-(1-piperidinylmethyl)phenoxy]propyl]-1,2-hydrazindicarboxamid

Die Herstellung erfolgt analog Beispiel 20 aus N-[3-[3-(1-Piperidinylmethyl)phenoxy]propyl]hydrazincarboxamid und Isopropylisocyanat. Die Analysenwerte sind in Tabelle I zusammengestellt.

B e i s p i e l 23

N-Cyclohexyl-N'-[3-[3-(1-piperidinylmethyl)phenoxy]propyl]-1,2-hydrazindicarboxamid

Die Herstellung erfolgt analog Beispiel 20 aus N-[3-[3-(1-Piperidinylmethyl)phenoxy]propyl]hydrazincarboxamid und Cyclohexylisocyanat. Die Analysenwerte sind in Tabelle I zusammengestellt.

Beispiel 24

N-Benzyl-N'-[3-[3-(1-piperidinylmethyl)phenoxy]propyl]-1,2-hydrazindicarboxamid

Die Herstellung erfolgt analog Beispiel 20 aus N-[3-[3-(1-Piperidinylmethyl)phenoxy]propyl]hydrazincarboxamid und Benzylisocyanat. Die Analysenwerte sind in Tabelle I zusammengestellt.

Beispiel 25

N-Phenyl-N'-[3-[3-(1-piperidinylmethyl)phenoxy]propyl]-1,2-hydrazindicarboxamid

Die Herstellung erfolgt analog Beispiel 20 aus N-[3-[3-(1-Piperidinylmethyl)phenoxy]propyl]hydrazincarboxamid und Phenylisocyanat. Die Analysenwerte sind in Tabelle I zusammengestellt.

Beispiel 26

N-4-Chlorphenyl-N'-[3-[3-(1-piperidinylmethyl)phenoxy]propyl]-1,2-hydrazindicarboxamid

Die Herstellung erfolgt analog Beispiel 20 aus N-[3-[3-(1-Piperidinylmethyl)phenoxy]propyl]hydrazincarboxamid und 4-Chlorphenylisocyanat. Die Analysenwerte sind in Tabelle I zusammengestellt.

Tabelle I  Kenndaten der Ausgangsverbindungen der Beispiele 5 bis 26 der allgemeinen Formel

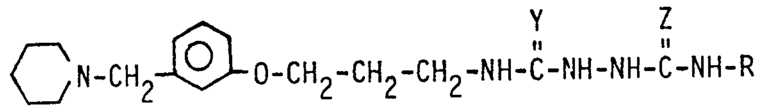

| Bei-spiel | R | Y<br>Z | Summenformel<br>MG | Analyse: Berechnet/Gefunden<br>C | H | N | Schmp.<br>°C | Ausbeute % d.Th. |
|---|---|---|---|---|---|---|---|---|
| 5 | $CH_3$ | S<br>S | $C_{18}H_{29}N_5OS_2$<br>395,6 | 54,7<br>54,7 | 7,39<br>7,23 | 17,7<br>17,9 | 154-6 | 78 |
| 6 | $C_2H_5$ | S<br>S | $C_{19}H_{31}N_5OS_2$<br>409,6 | 55,7<br>55,6 | 7,63<br>7,58 | 17,1<br>17,0 | 145-6 | 50 |
| 7 | $c-C_6H_{11}$ | S<br>S | $C_{23}H_{37}N_5OS_2$<br>463,7 | 59,6<br>59,4 | 8,04<br>8,08 | 15,1<br>15,2 | 171-2 | 43 |
| 8 | $COC_6H_5$ | S<br>S | $C_{24}H_{31}N_5O_2S_2$<br>x HCl 522,1 | 55,2<br>55,3 | 6,18<br>6,26 | 13,4<br>13,7 | 175-7 | 79 |
| 9 | $CH_3$ | S<br>O | $C_{18}H_{29}N_5O_2S$<br>379,5 | 57,0<br>56,8 | 7,70<br>7,73 | 18,5<br>18,2 | 187-8 | 88 |
| 10 | $C_2H_5$ | S<br>O | $C_{19}H_{31}N_5O_2S$<br>393,6 | 58,0<br>58,1 | 7,94<br>7,95 | 17,8<br>17,9 | 180-1 | 84 |

Tabelle I (Fortsetzung)

| | | | | | | | | |
|---|---|---|---|---|---|---|---|---|
| 11 | $CH(CH_3)_2$ | S<br>O | $C_{20}H_{33}N_5O_2S$<br>407,6 | 58,9<br>58,8 | 8,16<br>8,06 | 17,2<br>16,9 | 205-6 | 84 |
| 12 | $C_4H_9$ | S<br>O | $C_{21}H_{35}N_5O_2S$<br>421,6 | 59,8<br>59,8 | 8,37<br>8,25 | 16,6<br>16,3 | 192-3 | 92 |
| 13 | $c-C_6H_{11}$ | S<br>O | $C_{23}H_{37}N_5O_2S$<br>447,7 | 61,7<br>61,3 | 8,33<br>8,19 | 15,6<br>15,6 | 208-9 | 82 |
| 14 | $CH_2-C_6H_5$ | S<br>O | $C_{24}H_{33}N_5O_2S$<br>455,6 | 63,3<br>63,3 | 7,30<br>7,34 | 15,4<br>15,7 | 181-2 | 94 |
| 15 | $C_6H_5$ | S<br>O | $C_{23}H_{31}N_5O_2S$<br>441,6 | 62,6<br>62,3 | 7,08<br>7,01 | 15,9<br>15,6 | 176-7 | 64 |
| 16 | $4Cl-C_6H_4$ | S<br>O | $C_{23}H_{30}ClN_5O_2S$<br>476,0 | 58,0<br>57,9 | 6,35<br>6,38 | 14,7<br>14,9 | 163-8<br>Zers. | 59 |
| 17 | $CH_3$ | O<br>S | $C_{18}H_{29}N_5O_2S$<br>379,5 | 57,0<br>56,9 | 7,70<br>7,54 | 18,5<br>18,3 | 196 | 60 |

Tabelle I (Fortsetzung)

| | | | | | | | | |
|---|---|---|---|---|---|---|---|---|
| 18 | $C_2H_5$ | O<br>S | $C_{19}H_{31}N_5O_2S$<br>393,6 | 58,0<br>58,5 | 7,94<br>8,24 | 17,8<br>17,8 | 186 | 56 |
| 19 | c-$C_6H_{11}$ | O<br>S | $C_{23}H_{37}N_5O_2S$<br>447,7 | 61,7<br>61,5 | 8,33<br>8,59 | 15,6<br>15,5 | 196 | 50 |
| 20 | $CH_3$ | O<br>O | $C_{18}H_{29}N_5O_3$<br>363,5 | 59,5<br>59,4 | 8,04<br>7,81 | 19,3<br>19,3 | 233<br>Zers. | 67 |
| 21 | $C_2H_5$ | O<br>O | $C_{19}H_{31}N_5O_3$<br>377,5 | 60,5<br>60,2 | 8,28<br>8,23 | 18,6<br>18,3 | 233 | 69 |
| 22 | $CH(CH_3)_2$ | O<br>O | $C_{20}H_{33}N_5O_3$<br>391,5 | 61,4<br>61,3 | 8,50<br>8,48 | 17,9<br>17,7 | 204<br>Zers. | 67 |
| 23 | c-$C_6H_{11}$ | O<br>O | $C_{23}H_{37}N_5O_3$<br>431,6 | 64,0<br>63,9 | 8,64<br>8,54 | 16,2<br>16,2 | 199 | 50 |
| 24 | $CH_2C_6H_5$ | O<br>O | $C_{24}H_{33}N_5O_3$<br>439,6 | 65,6<br>65,6 | 7,57<br>7,40 | 15,9<br>15,9 | 211 | 70 |

Tabelle I (Fortsetzung)

| | | | | | | | | |
|---|---|---|---|---|---|---|---|---|
| 25 | $C_6H_5$ | 0 0 | $C_{23}H_{31}N_5O_3$ 425,5 | 64,9 65,0 | 7,34 7,31 | 16,5 16,6 | 206 Zers. | 60 |
| 26 | 4-Cl-$C_6H_4$ | 0 0 | $C_{23}H_{30}ClN_5O_3$ 460,0 | 60,1 60,2 | 6,57 6,47 | 15,2 15,0 | 203 | 60 |

0180754

## Beispiel 27

N,N-Dimethyl-N'-[3-[3-(1-piperidinylmethyl)phenoxy]propyl]-1,2-hydrazindicarboxamid

1,2 g (4 mmol) N-[3-[3-(1-piperidinylmethyl)phenoxy]propyl]hydrazincarboxamid in 20 ml Ether werden mit 0,6 ml (4 mmol) Triethylamin und 0,4 ml (4 mmol) N,N-Dimethylcarbamidchlorid versetzt und 12 Stunden gerührt. Festes Triethylammoniumchlorid wird abfiltriert und das Filtrat im Vakuum eingeengt.

Ausbeute: 1,2 g amorpher Feststoff (80% d.Th.)
$C_{19}H_{31}N_5O_3$ (377,5)

1H-NMR ($CDCl_3$; TMS): δ = 1,3-1,7 (m) ($-(CH_2)_3-$) 6H; 1,90 (m) ($CH_2$) 2H; 2,2-2,5 (m) ($-(CH_2)_2-$) 4H; 2,87 (s) ($(CH_3)_2$) 6H; 3,1-3,7 (m) (N-$CH_2$, NH (austauschbar mit $D_2O$)) 3H; 3,57 (s) ($CH_2$) 2H; 3,97 (t) (O-$CH_2$) 2H; 6,27 (t) (NH (austauschbar mit $D_2O$)) 1H; 6,6-7,3 (m) (Aromaten-H) 4H; 7,57 (s) (NH (austauschbar mit $D_2O$)) 1H.

## Beispiel 28

N,N-Dimethyl-2[[3-[3-(1-piperidinylmethyl)phenoxy]proyl]aminothioxomethyl]hydrazincarboxamid

1,62 g (5 mmol) N-[3-[3-(1-Piperidinylmethyl)phenoxy]propyl]hydrazincarbothioamid in 30 ml THF werden mit 1 ml Triethylamin und 0,6 g (6 mmol) N,N-Dimethylcarbamidchlorid versetzt und 3 Stunden unter Rückfluß erhitzt. Festes Triethylammoniumchlorid wird abfiltriert, das Filtrat eingedampft und der ölige Rückstand mit Ether zur Kristallisation gebracht.

Ausbeute: 1,5 g (76% d.Th.); Schmp.: 91-94°C (Ethanol/Wasser)
$C_{19}H_{31}N_5O_2S$ (393,6)

| | | | | |
|---|---|---|---|---|
| Berechnet: | C | 58,0 | H 7,94 | N 17,8 |
| Gefunden: | C | 58,1 | H 7,82 | N 17,6 |

0180754

Beispiel 29

N-Phenyl-N'-[3-[3-(1-piperidinylmethyl)phenoxy]propyl]-1,3,4-oxadiazol-2,5-diamin

1,5 g (5 mmol) N-[3-[3-(1-Piperidinylmethyl)phenoxy]propyl]hydrazincarboxamid in 30 ml THF werden mit 1,4 ml (10 mmol) Triethylamin und 0,7 ml (5 mmol) Phenylisocyaniddichlorid versetzt und bis zur quantitativen Umsetzung bei Raumtemperatur gerührt. Festes Triethylammoniumchlorid wird abfiltriert, das Filtrat im Vakuum eingedampft und der feste Rückstand aus Ethanol umkristallisiert. Die Analysenwerte sind in Tabelle II zusammengestellt.

Beispiel 30

N-Benzyl-N'-[3-[3-(1-piperidinylmethyl)phenoxy]propyl]-1,3,4-oxadiazol-2,5-diamin

Die Herstellung erfolgt analog Beispiel 29 aus N-[3-[3-(1-Piperidinylmethyl)phenoxy]propyl]hydrazincarboxamid und Benzylisocyaniddichlorid. Die Analysenwerte sind in Tabelle II zusammengestellt.

Beispiel 31

N-Cyclohexyl-N'-[3-[3-(1-piperidinylmethyl)phenoxy]propyl]-1,3,4-oxadiazol-2,5-diamin

Die Herstellung erfolgt analog Beispiel 29 aus N-[3-[3-(1-Piperidinylmethyl)phenoxy]propyl]hydrazincarboxamid und Cyclohexylisocyaniddichlorid. Die Analysenwerte sind in Tabelle II zusammengestellt.

Beispiel 32

N-Phenyl-N'-[3-[3-(1-piperidinylmethyl)phenoxy]propyl]-1,3,4-thiadiazol-2,5-diamin

Die Herstellung erfolgt analog Beispiel 29 aus N-[3-[3-(1-Piperidinyl-

methyl)phenoxy]propyl]hydrazincarbothioamid und Phenylisocyaniddichlorid. Die Analysenwerte sind in Tabelle II zusammengestellt.

Beispiel 33

N-Benzyl-N'-[3-[3-(1-piperidinylmethyl)phenoxy]propyl]-1,3,4-thiadiazol-2,5-diamin

Die Herstellung erfolgt analog Beispiel 29 aus N-[3-[3-(1-Piperidinylmethyl)phenoxy]propyl]hydrazincarbothioamid und Benzylisocyaniddichlorid. Die Analysenwerte sind in Tabelle II zusammengestellt.

Tabelle II Kenndaten der Verbindungen der Beispiele 29 bis 33 der allgemeinen Formel

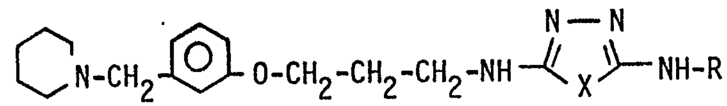

| Bei-spiel | X<br>R | Summenformel<br>MG | Analyse: Berechnet/Gefunden<br>C | H | N | Schmp.<br>°C | Ausbeute<br>% d.Th. |
|---|---|---|---|---|---|---|---|
| 29 | O<br>$C_6H_5$ | $C_{23}H_{29}N_5O_2$<br>407,5 | 67,8<br>67,6 | 7,17<br>6,90 | 17,2<br>16,9 | 144 Zers.<br>EtOH | 69 |
| 30 | O<br>$CH_2C_6H_5$ | $C_{24}H_{31}N_5O_2$<br>421,5 | 68,4<br>68,3 | 7,41<br>7,44 | 16,6<br>16,7 | 131-2<br>EtOH | 50 |
| 31 | O<br>$c\text{-}C_6H_{11}$ | $C_{23}H_{35}N_5O_2$<br>x HCl 450,0 | 61,4<br>61,4 | 8,06<br>8,16 | 15,6<br>15,8 | 208 Zers.<br>MeOH | 67 |
| 32 | S<br>$C_6H_5$ | $C_{23}H_{29}N_5OS$<br>423,6 | 65,2<br>65,2 | 6,90<br>6,94 | 16,5<br>16,5 | 141-3<br>EtOH | 75 |
| 33 | S<br>$CH_2C_6H_5$ | $C_{24}H_{31}N_5OS$<br>437,6 | 65,9<br>66,0 | 7,14<br>7,22 | 16,0<br>16,4 | 87-8<br>ACN | 42 |

Beispiel 34

N-[2-Methyl-3-[3-(1-piperidinylmethyl)phenoxy]propyl]-N'-phenyl-1,3,4-oxadiazol-2,5-diamin

0,96 g (3 mmol) N-[2-Methyl-3-[3-(1-piperidinylmethyl)phenoxy]propyl]-hydrazincarboxamid werden analog Beispiel 29 umgesetzt und isoliert.

Ausbeute: 0,61 g (48% d.Th.); Schmp.: 116°C (Acetonitril)

$C_{24}H_{31}N_5O_2$ (421,5)

| | | | |
|---|---|---|---|
| Berechnet: | C 68,4 | H 7,41 | N 16,6 |
| Gefunden: | C 68,0 | H 7,22 | N 16,6 |

Beispiel 35

N-[2-Hydroxy-3-[3-(1-piperidinylmethyl)phenoxy]propyl]-N'-phenyl-1,3,4-thiadiazol-2,5-diamin

0,68 g (2 mmol) N-[2-Hydroxy-3-[3-(1-piperidinylmethyl)phenoxy]propyl]-hydrazincarbothioamid werden analog Beispiel 29 umgesetzt und isoliert.

Ausbeute: 0,48 g (55% d.Th.); Schmp.: 150-152°C (Acetonitril)

$C_{23}H_{29}N_5O_2S$ (439,6)

| | | | |
|---|---|---|---|
| Berechnet: | C 62,8 | H 6,65 | N 15,9 |
| Gefunden: | C 62,7 | H 6,57 | N 16,1 |

Beispiel 36

N-Methyl-N'-[3-[3-(1-piperidinylmethyl)phenoxy]propyl]-1,3,4-thiadiazol-2,5-diamin

1,58 g (4 mmol) N-Methyl-N'-[3-[3-(1-piperidinylmethyl)phenoxy]propyl]-1,2-hydrazindicarbothioamid in 30 ml Ethanol werden mit 2 ml 30%igem Wasserstoffperoxid versetzt und kurz aufgekocht. Der sich abscheidende Schwefel wird unter Verwendung von Aktivkohle heiß abfiltriert, das Filtrat eingedampft und durch präparative Schichtchromatographie gereinigt.

0180754

Ausbeute: 0,8 g farbloser Schaum (55% d.Th.)
$C_{18}H_{27}N_5OS$ (361,5)

1H-NMR (DMSO-d6, TMS): $\delta$ = 1,1-1,7 (m) ($-(CH_2)_3-$) 6H; 1,98 (m) ($-CH_2-$) 2H; 2,1-2,4 (m) ($-(CH_2)_2-$) 4H; 2,74 (d) ($N-CH_3$) 3H; 3,1-3,6 (m) ($N-CH_2-$) 2H; 3,35 (s) ($N-CH_2-$) 2H; 3,98 (t) ($O-CH_2-$) 2H; 6,5-7,4 (m) (Aromaten-H, $(NH)_2$ (austauschbar mit $D_2O$)) 6H.

## B e i s p i e l 37

N-Ethyl-N'-[3-[3-(1-piperidinylmethyl)phenoxy]propyl]-1,3,4-thiadiazol-2,5-diamin

1,64 g (4 mmol) N-Ethyl-N'-[3-[3-(1-piperidinylmethyl)phenoxy]propyl]-1,2-hydrazindicarbothioamid in 30 ml Ethanol werden analog Beispiel 36 umgesetzt und isoliert.
Ausbeute: 1,1 g farbloser Schaum (73% d.Th.); Schmp. 102 - 104°C
$C_{19}H_{29}N_5OS$ (375,5)

| | | | |
|---|---|---|---|
| Berechnet: | C 60,8 | H 7,78 | N 18,6 |
| Gefunden: | C 60,6 | H 7,87 | N 18,4 |

1H-NMR (DMSO-d6, TMS): $\delta$ = 1,13 (t) ($C-CH_3$) 3H; 1,1-1,7 (m)($-(CH_2)_3-$) 6H; 1,98 (m) ($-CH_2-$) 2H; 2,1-2,4 (m) ($-(CH_2)_2-$) 4H; 2,9-3,6 (m) ($(N-CH_2-)_2$) 4H; 3,35 (s) ($N-CH_2-$) 2H; 3,98 (t) ($O-CH_2-$) 2H; 6,5-7,4 (m) (Aromaten-H, $(NH)_2$ (austauschbar mit $D_2O$)) 6H.

Bei analoger Umsetzung von N-Cyclohexyl-N'-[3-[3-(1-piperidinylmethyl)-phenoxy]propyl]-1,2-hydrazindicarbothioamid erhält man eine Verbindung, die mit dem weiter unten beschriebenen Beispiel 42 identisch ist.

## B e i s p i e l 38

N-Benzoyl-N'-[3-[3-(1-piperidinylmethyl)phenoxy]propyl]-1,3,4-thiadiazol-2,5-diamin

2,61 g (5 mmol) N-Benzoyl-N'-[3-[3-(1-piperidinylmethyl)phenoxy]propyl]-1,2-hydrazindicarbothioamidhydrochlorid werden in 50 ml Ethanol unter

Erwärmen gelöst und mit 2 ml 30%igem Wasserstoffperoxid versetzt. Nach kurzem Aufkochen der Lösung wird der ausgeschiedene Schwefel abfiltriert, mit Ammoniaklösung das Produkt gefällt und aus Ethanol/Wasser umkristallisiert.

Ausbeute: 1,65 g (73% d.Th.); Schmp.: 184-186°C (Ethanol)
$C_{24}H_{29}N_5O_2S$ (451,6)

| | | | |
|---|---|---|---|
| Berechnet: | C 63,8 | H 6,47 | N 15,5 |
| Gefunden: | C 63,8 | H 6,45 | N 15,4 |

Beispiel 39

N-Benzoyl-N'-[2-hydroxy-3-[3-(1-piperidinylmethyl)phenoxy]propyl]-1,3,4-thiadiazol-2,5-diamin

0,68 g (2 mmol) N-[2-Hydroxy-3-[3-(1-piperidinylmethyl)phenoxy]propyl]-hydrazincarbothioamid in 20 ml THF werden mit 0,33 g (2 mmol) Benzoylisothiocyanat versetzt und bei Raumtemperatur über Nacht gerührt. Nach Verdampfen des THF wird der feste Rückstand mit Ether gewaschen, in 30 ml Ethanol unter Erhitzen gelöst und mit 1 ml 30%igem Wasserstoffperoxid versetzt. Der ausgeschiedene Schwefel wird abfiltriert, das Filtrat eingedampft und der ölige Rückstand durch präparative Schichtchromatographie gereinigt.

Ausbeute: 0,18 g farbloser Schaum (19% d.Th.)
$C_{24}H_{29}N_5O_3S$ (467,6)

Beispiel 40

N-(1-Methylethyl)-N'-[3-[3-(1-piperidinylmethyl)phenoxy]propyl]-1,3,4-thiadiazol-2,5-diamin

1,63 g (4 mmol) N-(1-Methylethyl)-2-[[3-[3-(1-piperidinylmethyl)phenoxy]-propyl]-aminothioxomethyl]hydrazincarboxamid werden in 10 ml Phosphoroxychlorid unter schwachem Rückfluß 1 Stunde gerührt. Der Reaktionsansatz wird dann auf 300 ml Eiswasser gegossen und mit konzentrierter Ammoniaklösung auf ca. pH 9 gebracht. Der entstehende Niederschlag wird abgetrennt, mit Wasser gewaschen und aus Acetonitril umkristallisiert. Die Analysenwerte sind in Tabelle III zusammengestellt.

Beispiel 41

N-Butyl-N'-[3-[3-(1-piperidinylmethyl)phenoxy]propyl]-1,3,4-thiadiazol-2,5-diamin

Die Herstellung erfolgt analog Beispiel 40 aus N-Butyl-2-[[3-[3-(1-piperidinylmethyl)phenoxy]propyl]aminothioxomethyl]hydrazincarboxamid und Phosphoroxychlorid. Die Analysenwerte sind in Tabelle III zusammengestellt.

Beispiel 42

N-Cyclohexyl-N'-[3-[3-(1-piperidinylmethyl)phenoxy]propyl]-1,3,4-thiadiazol-2,5-diamin

Die Herstellung erfolgt analog Beispiel 40 aus N-Cyclohexyl-2-[[3-[3-(1-piperidinylmethyl)phenoxy]propyl]aminothioxomethyl]hydrazincarboxamid und Phosphoroxychlorid. Die Analysenwerte sind in Tabelle III zusammengestellt.

Beispiel 43

N-4-Chlorphenyl-N'-[3-[3-(1-piperidinylmethyl)phenoxy]propyl]-1,3,4-thiadiazol-2,5-diamin

Die Herstellung erfolgt analog Beispiel 40 aus N-4-Chlorphenyl-N'-[[3-[3-(1-piperidinylmethyl)phenoxy]propyl]aminothioxomethyl]hydrazincarboxamid und Phosphoroxychlorid. Die Analysenwerte sind in Tabelle III zusammengestellt.

Beispiel 44

N-Methyl-N'-[3-[3-(1-piperidinylmethyl)phenoxy]propyl]-1,3,4-oxadiazol-2,5-diamin

Die Herstellung erfolgt analog Beispiel 40 aus N-Methyl-N'-[3-[3-(1-piperidinylmethyl)phenoxy]propyl]-1,2-hydrazindicarboxamid und Phosphoroxychlorid. Die Analysenwerte sind in Tabelle III zusammengestellt.

Beispiel 45

N-Ethyl-N'-[3-[3-(1-piperidinylmethyl)phenoxy]propyl]-1,3,4-oxadiazol-2,5-diamin

Die Herstellung erfolgt analog Beispiel 40 aus N-Ethyl-N'-[3-[3-(1-piperidinylmethyl)phenoxy]propyl]-1,2-hydrazindicarboxamid und Phosphoroxychlorid. Die Analysenwerte sind in Tabelle III zusammengestellt.

Beispiel 46

N-Isopropyl-N'-[3-[3-(1-piperidinylmethyl)phenoxy]propyl]-1,3,4-oxadiazol-2,5-diamin

Die Herstellung erfolgt analog Beispiel 40 aus N-Isopropyl-N'-[3-[3-(1-piperidinylmethyl)phenoxy]propyl]-1,2-hydrazindicarboxamid und Phosphoroxychlorid. Die Analysenwerte sind in Tabelle III zusammengestellt.

Beispiel 47

N-4-Chlorphenyl- N'-[3-[3-(1-piperidinylmethyl)phenoxy]propyl]-1,3,4-oxadiazol-2,5-diamin

Die Herstellung erfolgt analog Beispiel 40 aus N-4-Chlorphenyl-N'-[3-[3-(1-piperidinylmethyl)phenoxy]propyl]-1,2-hydrazindicarboxamid und Phosphoroxychlorid. Die Analysenwerte sind in Tabelle III zusammengestellt.

Tabelle III Kenndaten der erfindungsgemäßen Verbindungen der Beispiele 40 bis 47 allgemeinen Formel

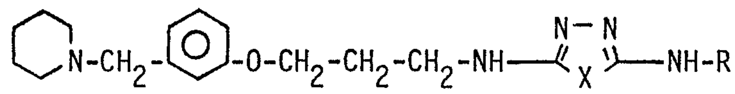

| Bei-spiel | X<br>R | Summenformel<br>MG | Analyse: Berechnet/Gefunden<br>C | H | N | Schmp.<br>°C | Ausbeute<br>% d.Th. |
|---|---|---|---|---|---|---|---|
| 40 | S<br>$CH(CH_3)_2$ | $C_{20}H_{31}N_5OS$<br>389,6 | 61,7<br>61,7 | 8,02<br>8,08 | 18,0<br>18,3 | 110-1 | 67 |
| 41 | S<br>$C_4H_9$ | $C_{21}H_{33}N_5OS$<br>403,6 | 62,5<br>62,5 | 8,24<br>8,16 | 17,4<br>17,3 | 98-100 | 70 |
| 42 | S<br>$c-C_6H_{11}$ | $C_{23}H_{35}N_5OS$<br>429,6 | 64,3<br>64,1 | 8,21<br>8,26 | 16,3<br>16,3 | 130-2 | 68 |
| 43 | S<br>$4-Cl-C_6H_4$ | $C_{23}H_{28}ClN_5OS$<br>458,0 | 60,3<br>60,4 | 6,16<br>6,21 | 15,3<br>15,3 | 174-6 | 52 |
| 44 | O<br>$CH_3$ | $C_{18}H_{27}N_5O_2$<br>x 0,5 $H_2O$<br>354,5 | 61,0<br>61,0 | 7,96<br>8,00 | 19,8<br>19,5 | 152<br>Zers. | 60 |

Tabelle III (Fortsetzung)

| | | | | | | | |
|---|---|---|---|---|---|---|---|
| 45 | 0<br>$C_2H_5$ | $C_{19}H_{29}N_5O_2$ x 0,5 $H_2O$<br>368,5 | 61,9<br>62,2 | 8,21<br>7,89 | 19,0<br>19,0 | 129 | 64 |
| 46 | 0<br>$CH(CH_3)_2$ | $C_{20}H_{31}N_5O_2$ x HCl x 0,5 $H_2O$<br>419,0 | 57,3<br>57,3 | 7,94<br>7,69 | 16,7<br>16,9 | 184 Zers. | 54 |
| 47 | 0<br>$4\text{-}Cl\text{-}C_6H_4$ | $C_{23}H_{28}ClN_5O_2$ x HCl x 0,5 $H_2O$<br>487,4 | 56,7<br>56,5 | 6,20<br>6,12 | 14,4<br>14,0 | 183 Zers. | 56 |

0180754

Beispiel 48

N,N-Dimethyl-N'-[3-[3-(1-piperidinylmethyl)phenoxy]propyl]-1,3,4-thiadiazol-2,5-diamin

1,18 g (3 mmol) N,N-Dimethyl-2-[[3-[3-(1-piperidinylmethyl)phenoxy]-propyl]-aminothioxomethyl]hydrazincarboxamid werden analog Beispiel 40 umgesetzt und isoliert.

Ausbeute: 0,8 g (71% d.Th.); Schmp.: 128-129°C (Ethanol/Wasser)
$C_{19}H_{29}N_5OS$ (375,5)

| | | | |
|---|---|---|---|
| Berechnet: | C 60,8 | H 7,78 | N 18,6 |
| Gefunden: | C 60,5 | H 7,76 | N 18,8 |

Beispiel 49

N,N-Dimethyl-N'-[3-[3-(1-piperidinylmethyl)phenoxy]propyl]-1,3,4-oxadiazol-2,5-diamin

1,13 g (3 mmol) N,N-Dimethyl-N'-[3-[3-(1-piperidinylmethyl)phenoxy]propyl]-1,2-hydrazindicarboxamid werden analog Beispiel 40 umgesetzt und isoliert.

Ausbeute: 0,65 g (60% d.Th.); Schmp.: 132°C zers. (Acetonitril)
$C_{19}H_{29}N_5O_2$ (359,5)

| | | | |
|---|---|---|---|
| Berechnet: | C 63,5 | H 8,13 | N 19,5 |
| Gefunden: | C 63,3 | H 8,10 | N 19,4 |

Beispiel 50

N-[3-[3-(1-piperidinylmethyl)phenoxy]propyl]-1,3,4-thiadiazol-2,5-diamin

0,9 g (2 mmol) N-Benzoyl-N'-[3-[3-(1-piperidinylmethyl)phenoxy]propyl]-1,3,4-thiadiazol-2,5-diamin werden in 20 ml konzentrierter Salzsäure 2 Stunden unter Rückfluß erhitzt. Nach Verdampfen der Salzsäure bis zur

Trockene wird der feste Rückstand zunächst mit Ether digeriert, dann in wenig Ethanol gelöst, mit konzentrierter Ammoniaklösung das Produkt gefällt und aus Ethanol/Wasser umkristallisiert.

Ausbeute: 0,5 g (70% d.Th.); Schmp.: 158-159°C (Ethanol/Wasser)
$C_{17}H_{25}N_5OS$ (347,5)

| | | | |
|---|---|---|---|
| Berechnet: | C 58,8 | H 7,25 | N 20,2 |
| Gefunden: | C 58,8 | H 7,28 | N 20,0 |

B e i s p i e l 51

N-[4-[3-(1-Piperidinylmethyl)phenoxy]-2-butenyl]-1,3,4-thiadiazol-2,5-diamin

0,6 g (2,3 mmol) 4-[3-(1-Piperidinylmethyl)phenoxy]-2-buten-1-amin, 0,35 ml Triethylamin und 0,42 g (2,3 mmol) 5-Brom-1,3,4-thiadiazol-2-amin werden in 20 ml THF 3 Stunden unter Rückfluß erhitzt. Festes Triethylammoniumbromid wird abfiltriert, das Filtrat eingedampft und der ölige Rückstand durch präparative Schichtchromatographie gereinigt.

Ausbeute: 0,25 g (30% d.Th.); Schmp. 131 - 134°C
$C_{18}H_{25}N_5OS$ (359,4)

| | | | |
|---|---|---|---|
| Berechnet: | C 60,1 | H 7,01 | N 19,5 |
| Gefunden: | C 60,2 | H 7,13 | N 19,6 |

1H-NMR ($CDCl_3$TMS): $\delta$ = 1,2-1,7 (m) ($-(CH_2)_3-$) 6H; 2,1-2,6 (m) ($-(CH_2)_2-$) 4H; 3,49 (s) ($N-CH_2-$) 2H; 3,6-4,0 (m) ($N-CH_2-$) 2H; 4,15-4,55 (m) ($O-CH_2-$) 2H; 5,83 (m) (CH=CH) 2H; 6,15 (br. s) ($NH_2$ (austauschbar mit $D_2O$)); 6,6-7,2 (m) (Aromaten-H, (NH) (austauschbar mit $D_2O$)) 5H.

B e i s p i e l 52

1-[(5-Amino-1,3,4-thiadiazol-2-yl)amino]-3-[3-(1-piperidinylmethyl)-phenoxy]-2-propanol

0,8 g (3 mmol) 1-Amino-3-[3-(1-piperidinylmethyl)phenoxy]-2-propanol in 20 ml THF werden mit 0,54 g (3 mmol) 5-Brom-1,3,4-thiadiazol-2-amin und 0,45 ml Triethylamin versetzt und analog Beispiel 23 umgesetzt. Das erhaltene Öl wird durch Säulenchromatographie gereinigt (Kieselgel; ammoniakal. Methanol-Chloroform-Lösung (5+95)).

Ausbeute: 0,3 g (28% d.Th.); Schmp.: 130 - 132° C
$C_{17}H_{25}N_5O_2S$ (363,5)

| | | | |
|---|---|---|---|
| Berechnet: | C 56,2 | H 6,93 | N 19,3 |
| Gefunden: | C 55,7 | H 6,96 | N 19,5 |

1H-NMR ($d_6$-DMSO,TMS) $\delta$ = 1.25-1.55 (m) (C-$(CH_2)_3$-C) 6 H; 2.30 (m) ($N(CH_2)_2$) 4 H; 3.32 (m) ($CH_2$-NH) 2 H; 3.37 (s) (N-$CH_2$) 2 H; 3.85-4.10 (m) (O-$CH_2$-CHOH) 3 H; 5.27 (d) 1 H; 6.24 (s) 2 H; 6.78-6.90 (m) 4 H; 7.21 (t) 1 H ppm.

## B e i s p i e l 53

(±)-N-[2-Methyl-3-[3-(1-piperidinylmethyl)phenoxy]propyl]-1,3,4-thiadiazol-2,5-diamin

Die Herstellung erfolgt analog Beispiel 51 aus 2-Methyl-3-[3-(1-piperidinylmethyl)phenoxy]-1-propanamin und 5-Brom-1,3,4-thiadiazol-2-amin.

Ausbeute: 74 % d.Th.; Schmp. 133 - 135° C
$C_{18}H_{27}N_5OS$ (361.5)

| | | | |
|---|---|---|---|
| Berechnet: | C 59.8 | H 7.53 | N 19.4 |
| Gefunden: | C 59.8 | H 7.49 | N 19.4 |

1H-NMR ($d_6$-DMSO, TMS): $\delta$ = 1.01 (d) ($CH_3$) 3 H; 1.25-1.55 (m) (C-$(CH_2)_3$-C) 6 H; 2.15-2.40 (m) ($N(CH_2)_2$, CH-$CH_3$) 5 H; 3.20 (m) ($CH_2$-NH) 2 H; 3.37 (s) (N-$CH_2$) 2 H; 3.85 (m) (O-$CH_2$) 2 H; 6.23 (s) 2 H; 6.78-6.90 (m) 4 H; 7.20 (t) 1 H, ppm.

Beispiel 54

N-[3-[3-[(Dimethylamino)methyl] phenoxy] propyl] -1,3,4-thiadiazol-2,5-diamin

Die Herstellung erfolgt analog Beispiel 51 aus 3-[3-[(Dimethylamino) methyl] phenoxy] -1-propanamin und 5-Brom-1,3,4-thiadiazol-2-amin.

Ausbeute: 38% d.Th.; Schmp. 130 - 131° C

$C_{14}H_{21}N_5OS$ (307.4)

| | | | |
|---|---|---|---|
| Berechnet: | C 54.7 | H 6.89 | N 22.8 |
| Gefunden: | C 54.7 | H 6.80 | N 22.9 |

1H-NMR ($d_6$-DMSO, TMS): $\delta$ = 1.97 (quin.) (O-$CH_2$-$CH_2$) 2 H; 2.13 (s) ($N(CH_3)_2$) 6 H; 3.29 (q) ($CH_2$-NH) 2 H; 3.34 (s) (N-$CH_2$) 2 H; 4.01 (t) (O-$CH_2$) 2 H; 6.24 (s) 2 H; 6.80-6.90 (m) 4 H; 7.22 (t) 1 H ppm.

Beispiel 55

N-[3-[3-(1-Pyrrolidinylmethyl)phenoxy]propyl]-1,3,4-thiadiazol-2,5-diamin

Die Herstellung erfolgt analog Beispiel 51 aus 3-[3-(1-Pyrrolidinylmethyl) phenoxy]-1-propanamin und 5-Brom-1,3,4-thiadiazol-2-amin.

Ausbeute: 66 % d.Th.; Schmp. 138 - 140° C

$C_{16}H_{23}N_5OS$ (333.5)

| | | | |
|---|---|---|---|
| Berechnet: | C 57.6 | H 6.95 | N 21.0 |
| Gefunden: | C 57.6 | H 6.75 | N 21.0 |

1H-NMR ($d_6$-DMSO, TMS): $\delta$ = 1.69 (m) (C-$(CH_2)_2$-C) 4 H; 1.97 (quin) (O-$CH_2$-$CH_2$) 2 H; 2.41 (m) ($N(CH_2)_2$) 4 H; 3.29 (q) ($CH_2$-NH) 2 H; 3.52 (s) (N-$CH_2$) 2 H; 4.01 (t) (O-$CH_2$) 2 H; 6.24 (s) 2 H; 6.75-6.90 (m) 4 H; 7.21 (t) 1 H ppm.

## Beispiel 56

N-[3-[3-(1-Piperidinylmethyl)phenoxy]propyl]-1,3,4-thiadiazol-2,5-diamin

Die Herstellung erfolgt analog Beispiel 51 aus 3-[3-(1-Piperidinylmethyl) phenoxy]-1-propanamin und 5-Brom-1,3,4-thiadiazol-2-amin. Das erhaltene Produkt ist identisch mit der in Beispiel 50 beschriebenen Substanz.

Ausbeute: 55 % d.Th.; Schmp. 158 - 159° C

## Beispiel 57

N-[3-[3-[(Hexahydro-1H-azepin-1-yl)methyl]phenoxy]propyl]-1,3,4-thiadiazol-2,5-diamin

Die Herstellung erfolgt analog Beispiel 51 aus 3-[3-[(Hexahydro-1H-azepin-1-yl)methyl]phenoxy]-1-propanamin und 5-Brom-1,3,4-thiadiazol-2-amin.

Ausbeute: 69 % d.Th.; Schmp. 140 - 142° C

$C_{18}H_{27}N_5OS$ (361.5)

| | | | |
|---|---|---|---|
| Berechnet: | C 59.8 | H 7.53 | N 19.4 |
| Gefunden: | C 59.7 | H 7.46 | N 19.6 |

1H-NMR ($d_6$-DMSO, TMS): $\delta$ = 1.56 (m) (C-$(CH_2)_4$-C) 8 H; 1.97 (quin) (O-$CH_2$-$CH_2$) 2 H; 2.55 (m) (N$(CH_2)_2$) 4 H; 3.29 (q) ($CH_2$-NH) 2 H; 3.55 (s) (N-$CH_2$) 2 H; 4.01 (t) (O-$CH_2$) 2 H; 6.24 (s) 2 H; 6.75-6.90 (m) 4 H; 7.21 (t) 1 H ppm.

## Beispiel 58

N-[3-[3-(4-Morpholinylmethyl)phenoxy]propyl]-1,3,4-thiadiazol-2,5-diamin

Die Herstellung erfolgt analog Beispiel 51 aus 3-[3-(4-Morpholinylmethyl) phenoxy]-1-propanamin und 5-Brom-1,3,4-thiadiazol-2-amin.

0180754

Ausbeute: 60 % d.Th.; Schmp. 170 - 172° C
$C_{16}H_{23}N_5O_2S$ (349.5)

| | | | |
|---|---|---|---|
| Berechnet: | C 55.0 | H 6.63 | N 20.0 |
| Gefunden: | C 54.9 | H 6.57 | N 19.8 |

1H-NMR ($d_6$-DMSO, TMS): δ = 1.96 (quin) (O-$CH_2$-$CH_2$) 2 H; 2.33 (m) (N$(CH_2)_2$) 4 H; 3.29 (q) ($CH_2$-NH) 2 H; 3.41 (s) (N-$CH_2$) 2 H; 3.57 (m) (O$(CH_2)_2$) 4 H; 4.01 (t) (O-$CH_2$) 2 H; 6.25 (s) 2 H; 6.79-6.88 (m) 4 H; 7.22 (t) 1 H ppm.

## B e i s p i e l 59

N-[3-[3-[(4-Methyl-1-piperazinyl)methyl]phenoxy]propyl]-1,3,4-thiadiazol-2,5-diamin

Die Herstellung erfolgt analog Beispiel 51 aus 3-[3-[(4-Methyl-1-piperazinyl)methyl]phenoxy]-1-propanamin und 5-Brom-1,3,4-thiadiazol-2-amin.

Ausbeute: 63 % d.Th.; Schmp. 146 - 148° C
$C_{17}H_{26}N_6OS$ (362.5)

| | | | |
|---|---|---|---|
| Berechnet: | C 56.3 | H 7.22 | N 23.2 |
| Gefunden: | C 56.4 | H 7.03 | N 23.4 |

1H-NMR ($d_6$-DMSO, TMS): δ = 1.97 (quin) (O-$CH_2$-$CH_2$) 2 H; 2.14 (s) (N-$CH_3$) 3 H; 2.20-2.40 (m) (Piperazin) 8 H; 3.29 (q) ($CH_2$-NH) 2 H; 3.40 (s) (N-$CH_2$) 2 H; 4.01 (t) (O-$CH_2$) 2 H; 6.24 (s) 2 H; 6.78-6.87 (m) 4 H; 7.21 (t) 1 H ppm.

## B e i s p i e l 60

N-[3-[3-(1-Piperidinylmethyl)phenoxy]propyl]-1,3,4-oxadiazol-2,5-diamin

Die Herstellung erfolgt analog Beispiel 40 aus N-[3-[3-(1-Piperidinylmethyl)phenoxy]propyl]-1,2-hydrazindicarboxamid und Phosphoroxychlorid.

Ausbeute: 25 % d.Th.; Schmp. 114° C
$C_{17}H_{25}N_5O_2$ (331.4)

| | | | |
|---|---|---|---|
| Berechnet: | C 61.6 | H 7.60 | N 21.1 |
| Gefunden: | C 61.4 | H 7.87 | N 20.9 |

1H-NMR ($d_6$-DMSO, TMS): δ = 1.25-1.55 (m) (C-$(CH_2)_3$-C) 6 H; 1.96 (quin) (O-$CH_2$-$CH_2$) 2 H; 2.30 (m) (N$(CH_2)_2$) 4 H; 3.21 (q) ($CH_2$-NH) 2 H; 3.37 (s) (N-$CH_2$) 2 H; 4.00 (t) (O-$CH_2$) 2 H; 6.29 (s) 2 H; 6.75-6.90 (m) 4 H; 7.21 (t) 1 H ppm.

## Beispiel 61

(±)-N-[2-Methyl-3-[3-(1-piperidinylmethyl)phenoxy]propyl]-1,3,4-oxadiazol-2,5-diamin

7 mmol N-[2-Methyl-3-[3-(1-piperidinylmethyl)phenoxy]propyl]-1,2-hydrazindicarboxamid werden in 30 ml Phosphoroxychlorid 6 h bei 50 - 60° C gerührt. Der abgekühlte Ansatz wird auf 300 ml Eiswasser gegossen und unter Kühlung mit konz. Ammoniaklösung neutralisiert. Das Produkt wird mit Ether extrahiert, die vereinigten Etherextrakte gewaschen, getrocknet und eingedampft. Der feste Rückstand wird aus Ether umkristallisiert.

Ausbeute: 20% d.Th.; Schmp. 92° C
$C_{18}H_{27}N_5O_2$ (345.5)

| | | | |
|---|---|---|---|
| Berechnet: | C 62.6 | H 7.88 | N 20.3 |
| Gefunden: | C 62.5 | H 7.72 | N 20.2 |

1H-NMR ($CDCl_3$, TMS): δ = 1.08 (d) ($CH_3$) 3 H; 1.25-1.55 (m) (C-$(CH_2)_3$-C) 6 H; 2.10-2.50 (m) (N$(CH_2)_2$,CH-$CH_3$) 5 H; 3.15-3.50 (m) ($CH_2$-NH, N-$CH_2$) 4 H; 3.65-4.00 (m) (O-$CH_2$) 2 H; 4.50-5.20 (3H); 6.60-7.30 (m) 4 H ppm.

## Beispiel 62

N-[3-[3-(4-Thiomorpholinylmethyl)phenoxy]propyl]-1,3,4- thiadiazol-2,5-diamin

Die Herstellung erfolgt analog Beispiel 51 aus 3-[3-(4-Thiomorpholinylmethyl)phenoxy]-1-propanamin und 5-Brom-1,3,4-thiadiazol-2-amin.

Ausbeute: 58 % d.Th.; Schmp. 158 - 160° C

$C_{16}H_{23}N_5OS_2$ (365.5)

| | | | |
|---|---|---|---|
| Berechnet: | C 52.6 | H 6.34 | N 19.2 |
| Gefunden: | C 52.5 | H 6.32 | N 19.4 |

1H-NMR ($d_6$-DMSO, TMS): δ = 1.97 (quin) ($CH_2$) 2 H; 2.60 (s) ($-(CH_2)_4-$) 8 H; 3.29 (q) ($CH_2$) 2 H; 3.45 (s) ($CH_2$) 2 H; 4.01 (t) ($CH_2$) 2 H; 6.25 (s) 2 H; 6.80-6.89 (m) 4 H; 7.22 (t) 1 H ppm.

## Beispiel 63

N-[4-[3-(1-Piperidinylmethyl)phenoxy]butyl]-1,3,4-thiadiazol-2,5-diamin

Die Herstellung erfolgt analog Beispiel 51 aus 4-[3-(1-Piperidinylmethyl)phenoxy]-1-butanamin und 5-Brom-1,3,4-thiadiazol-2-amin.

Ausbeute: 61 % d.Th.; Schmp. 139° C

$C_{18}H_{27}N_5OS$ (361.5)

| | | | |
|---|---|---|---|
| Berechnet: | C 59.8 | H 7.53 | N 19.4 |
| Gefunden: | C 59.9 | H 7.45 | N 19.5 |

1H-NMR ($d_6$-DMSO, TMS): δ = 1.20-1.67 (m) ($-(CH_2)_3-$) 6 H; 1.73 (m) ($-(CH_2)_2-$) 4 H; 2.33 (m) ($-(CH_2)_2-$) 4 H; 3.20 (m) ($-CH_2-$, N-H) 3 H; 3.40 (s) ($-CH_2-$) 2 H; 4.00 (t) ($-O-CH_2-$) 2 H; 6.17 (s) ($-NH_2$) 2 H; 6.67-7.40 (m) 4 H ppm.

0180754

Beispiel 64

N-[3-[3-(3-Methylpiperidin-1-yl-methyl)phenoxy]propyl]-1,3,4-thiadiazol-2,5-diamin

Herstellung der Vorstufen

a) 3-[(3-Methylpiperidin-1-yl)methyl]phenol

24.4 g (0.2 mol) 3-Hydroxybenzaldehyd werden mit 40 ml (ca. 0,4 mol) 3-Methylpiperidin und 20 ml (ca. 0,52 mol) 100 proz. Ameisensäure unter Kühlung gemischt und anschließend 2 h bei 120° C gerührt. Der auf Raumtemperatur abgekühlte Reaktionsansatz wird in Wasser gegossen, mit Ammoniak alkalisiert, der ausgefallene Feststoff abfiltriert und aus Ethanol umkristallisiert.

Ausbeute: 37 g (90 % d.Th.); Schmp. 155 - 157° C

b) 3-[3-[(3-Methylpiperidin-1-yl)methyl]phenoxy]propanamin

Zu einer Suspension von 1.0 g (0.025 mol) Natriumhydrid (60 %) in abs. DMF werden 5.13 g (0.025 mol) 3-[(3-Methylpiperidin-1-yl)methyl]phenol eingetragen und über Nacht bei Raumtemperatur gerührt. Nach Zusatz von 2.23 g (0.025 mol) 3-Chlorpropionsäurenitril wird 7 h bei 60° C gerührt, anschließend in Eiswasser gegossen, mit Ether extrahiert und die organische Phase mit 5-proz. NaOH gewaschen. Die Etherphase wird nach Trocknen über $Na_2SO_4$ in eine Suspension von 1.14 g (0.03 mol) $LiAlH_4$ in abs. Ether getropft und anschließend mit 2.2 ml $H_2O$ hydrolysiert. Nach Abfiltrieren und Einengen des Lösungsmittels i. Vak. verbleiben 2.96 g eines hellgelben Öls.

N-[3-[3-(3-Methylpiperidin-1-yl-methyl)phenoxy]propyl]-1,3,4-thiadiazol-2,5-diamin

Die Herstellung erfolgt analog Beispiel 51 aus 3-[3-[(3-Methylpiperidin-1-yl)methyl]phenoxy]propanamin und 5-Brom-1,3,4-thiadiazol-2-amin.

Ausbeute: 45 % d.Th.; Schmp. 138,5 - 140,0° C
$C_{18}H_{27}N_5OS$ (361.5)

| | | | |
|---|---|---|---|
| Berechnet: | C 59.8 | H 7.48 | N 19.39 |
| Gefunden: | C 59.7 | H 7.45 | N 19.11 |

1H-NMR ($d_6$-DMSO, TMS): δ = 0.8 (d) 3 H; 1.28-2.16 (m) 10 H; 2.58-2.8 (m) 2 H; 3.27 (t) 2 H; 3.4 (s) 2 H; 4.03 (t) 2 H; 6.27 (s) 2 H; 6.7-7.37 (m) 4 H ppm.

## Beispiel 65

N-[4-[3-(3-Methylpiperidin-1-yl-methyl)phenoxy]butyl]-1,3,4-thiadiazol-2,5-diamin

Herstellung der Vorstufe

a) 4-[3-[(3-Methylpiperidin-1-yl)methyl] phenoxy]butanamin

Zu einer Suspension von 1,0 g (0,025 mol) Natriumhydrid (60 %) in abs. DMF werden 5,13 g (0,025 mol) 3-((-Methylpiperidin-1-yl)methyl)phenol (Beispiel 64 a) eingetragen und über Nacht bei Raumtemperatur gerührt. Nach Zusatz von 2,58 g (0,025 mol) 4-Chlorbutyronitril wird 7 h bei 60° C gerührt, anschließend in Eiswasser gegossen, mit Ether extrahiert und die organische Phase mit 5-proz. NaOH gewaschen. Die Etherphase wird nach Trocknen über $Na_2SO_4$ in eine Suspension von 1,14 g (0,03 mol) $LiAlH_4$ in abs. Ether getropft und anschließend mit 2,2 ml $H_2O$ hydrolysiert. Nach Abfiltrieren der Anorganika und Abziehen des Lösungsmittels i. Vak. verbleiben 3,66 g eines hellgelben Öls.

N-[4-[3-((3-Methylpiperidin-1-yl)methyl)phenoxy]butyl]-1,3,4-thiadiazol-2,5-diamin

Die Herstellung erfolgt analog Beispiel 51 aus 4-[3-[(3-Methylpiperidin-1-yl)methyl]phenoxy]-butanamin und 5-Brom-1,3,4-thiadiazol-2-amin.

Ausbeute: 54 % d.Th.; Schmp. 123 - 124° C
$C_{19}H_{29}N_5OS$ (375,5)

| | | | |
|---|---|---|---|
| Berechnet: | C 60,8 | H 7,78 | N 18,6 |
| Gefunden: | C 60,8 | H 7,78 | N 18,7 |

1H-NMR ($CDCl_3$; TMS) $\delta$ = 0.82-0.96 (m) 4 H; 1.52-1.76 (m) 5 H; 1.82-1.94 (m) 5 H; 2.52 (m) 2 H; 3.36 (dt) 2 H; 3.46 (s) 2 H; 4.0 (t) 2 H; 5.02 (s) 2 H; 5.42 (s) 1 H; 6,8 (m) 1 H; 6,92 (m) 2 H; 7.22 (m) 1 H ppm.

Beispiel 66

N-[3-[3-((3-Methylpyrrolidin-1-yl)methyl)phenoxy]propyl]-1,3,4-thiadiazol-2,5-diamin

Herstellung der Vorstufen

a) 3-[(3-Methylpyrrolidin-1-yl)methyl]phenol

Die Herstellung erfolgt analog Beispiel 64 a aus 3-Methyl-pyrrolidin.

Ausbeute: 85 % d.Th. (gelbes Öl)

b) 3-[3-[(3-Methylpyrrolidin-1-yl)methyl]phenoxy]propanamin

28,7 g (0,15 mol) 3-[(3-Methylpyrrolidin-1-yl)methyl]phenol werden mit 100 ml Acrylnitril und 1,5 ml 40proz. methanol. Benzyltrimethylammoniumhydroxid-Lösung 18 h unter Rückfluß erhitzt. Das überschüssige Acrylnitril wird i. Vak. entfernt, der Rückstand in Ether aufgenommen, mit 5-proz. NaOH gewaschen und mit 7,6 g (0,2 mol) $LiAlH_4$ analog Beispiel 64 b zum Amin reduziert. Man erhält 14,9 g (40 % d.Th.) eines hellgelben Öls, das für weitere Umsetzungen rein genug ist.

N-[3-[3-((3-Methylpyrrolidin-1-yl)methyl)phenoxy]propyl]-1,3,4-thiadiazol-2,5-diamin

Die Herstellung erfolgt analog Beispiel 51 aus 3-[3-[(3-Methylpyrrolidin-1-yl)methyl]phenoxy]propanamin und 5-Brom-1,3,4-thiadiazol-2-amin.

Ausbeute: 48 % d.Th.; Schmp. 116 - 119° C

$C_{17}H_{25}N_5OS$ (347,5)

| | | | |
|---|---|---|---|
| Berechnet: | C 58,8 | H 7,25 | N 20,2 |
| Gefunden: | C 58,7 | H 7,24 | N 20,1 |

1H-NMR ($CDCl_3$, TMS): $\delta$ = 1.0 (d) 3 H; 1.24 (m) 1 H; 1.96-2.34 (m) 5 H; 2.46 (m) 1 H; 2.74 (m) 1 H; 2.84 (m) 1 H; 3,5 (dt) 2 H; 3.56 (s) 2 H; 4.08 (t) 2 H; 4,8 (s) 2 H; 5.36 (s) 1 H; 6.79 (m) 1 H; 6.91 (m) 2 H; 7.21 (m) 1 H ppm.

## Beispiel 67

N-[4-[3-((3-Methylpyrrolidin-1-yl)methyl)phenoxy]butyl]-1,3,4-thiadiazol-2,5-diamin

Herstellung der Vorstufe

a) 4-[3-[(3-Methylpyrrolidin-1-yl)methyl]phenoxy]-butanamin

Die Darstellung erfolgt analog Beispiel 65 a aus 3-[(3-Methylpyrrolidin-1-yl)methyl]phenol. Man erhält ein hellgelbes Öl, das für weitere Umsetzungen rein genug ist.

Ausbeute: 60% d.Th.

N-[4-[3-((3-Methylpyrrolidin-1-yl)methyl)phenoxy]butyl]-1,3,4-thiadiazol-2,5-diamin

Die Herstellung erfolgt analog Beispiel 51 aus 4-[3-[(3-Methylpyrrolidin-1-yl)methyl]phenoxy]-butanamin und 5-Brom-1,3,4-thiadiazol-2-amin.

Ausbeute: 50 % d.Th.; Schmp. 118 - 119° C

$C_{18}H_{27}N_5OS$ (361,5)

| | | | |
|---|---|---|---|
| Berechnet: | C 59,8 | H 7,52 | N 19,4 |
| Gefunden: | C 59,8 | H 7,66 | N 19,3 |

1H-NMR ($CDCl_3$, TMS): $\delta$ = 1.0 (d) 3 H; 1.26-1.4 (m) 1 H; 1.78-1.92 (m) 4 H; 1.94-2.1 (m) 2 H; 2.18-2.34 (m) 1 H; 2.38-2.5 (m) 1 H; 2.68-2.78 (m) 1 H; 2.8-2.88 (m) 1 H; 3.36 (dt) 2 H; 3.58 (s) 2 H; 4.0 (t) 2 H; 4.9 (s) 2 H; 5.28 (s) 1 H; 6.78 (m) 1 H; 6.9 (m) 2 H; 7.22 (m) 1 H ppm.

## Beispiel 68

N-[3-[3-[(2,6-Dimethyl-1-piperidinyl)methyl]phenoxy]propyl]-1,3,4-thiadiazol-2,5-diamin

Die Herstellung erfolgt analog Beispiel 51 aus 3-[3-[(2,6-Dimethyl-1-piperidinyl)methyl]phenoxy]-1-propanamin und 5-Brom-1,3,4-thiadiazol-2-amin.

Ausbeute: 43 % d.Th.; Schmp. 149 - 151° C
$C_{19}H_{29}N_5OS$ (375.5)

| | | | |
|---|---|---|---|
| Berechnet: | C 60.8 | H 7.78 | N 18.6 |
| Gefunden: | C 60.7 | H 7.64 | N 18.7 |

1H-NMR($d_6$-DMSO, TMS): $\delta$ = 0.94 (d) ($CH_3$) 6 H; 1.15-1.65 (m) ($(CH_2)_3$) 6 H; 1.97 (quin) ($CH_2$) 2 H; 2.43 (m) ($N(CH_2)_2$ 2 H; 3.29 (q) ($CH_2$) 2 H; 3.66 (s) (N-$CH_2$) 2 H; 4.00 (t) ($CH_2$) 2 H; 6.24 (s) 2 H; 6.73-6.95 (m) 4 H; 7.18 (t) 1 H ppm.

## Beispiel 69

N-[3-[3-[(3,5-Dimethyl-1-piperidinyl)methyl]phenoxy]propyl]-1,3,4-thiadiazol-2,5-diamin

Die Herstellung erfolgt analog Beispiel 51 aus 3-[3-[(3,5-Dimethyl-1-piperidinyl)methyl]phenoxy]-1-propanamin und 5-Brom-1,3,4-thiadiazol-2-amin.

Ausbeute: 46 % d. Th.; Schmp. 112 - 116° C

$C_{19}H_{29}N_5OS$ (375.5)

| | | | |
|---|---|---|---|
| Berechnet: | C 60.8 | H 7.78 | N 18.6 |
| Gefunden: | C 60.7 | H 7.77 | N 18.6 |

1H-NMR ($CDCl_3$, TMS): δ = 0.50-2.90 (m) 16 H; 3.45 (s) (N-$CH_2$) 2 H; 3.51 (t) ($CH_2$-NH) 2 H; 4.08 (t) (O-$CH_2$) 2 H; 4.60-4.90 2 H; 5.10-5.40 1 H; 6.74-6.93 (m) 3 H; 7.22 (t) 1 H ppm.

## Beispiel 70

N-[3-[3-[(4-Methyl-1-piperidinyl)methyl]phenoxy]propyl]-1,3,4-thiadiazol-2,5-diamin

Die Herstellung erfolgt analog Beispiel 51 aus 3-[3-[(4-Methyl-1-piperidinyl)methyl]phenoxy]-1-propanamin und 5-Brom-1,3,4-thiadiazol-2-amin.

Ausbeute: 64 % d.Th.; Schmp. 141 - 143° C

$C_{18}H_{27}N_5OS$ (361.5)

| | | | |
|---|---|---|---|
| Berechnet: | C 59.8 | H 7.53 | N 19.4 |
| Gefunden: | C 59.8 | H 7.29 | N 19.3 |

1H-NMR ($CDCl_3$, TMS): δ = 0.91 (d) ($CH_3$) 3 H; 1.15-2.00 (m) 7 H; 2.12 (quin) (O-$CH_2$-$CH_2$) 2 H; 2.84 (m) 2 H; 3.45 (s) (N-$CH_2$) 2 H; 3.51 (t) ($CH_2$-NH) 2 H; 4.08 (t) (O-$CH_2$) 2 H; 4.60-4.80 2 H; 5.10-5.40 1 H; 6.75-6.92 (m) 3 H; 7.21 (t) 1 H ppm.

## PATENTANSPRÜCHE

1. 3,4-Diazolderivate der allgemeinen Formel I

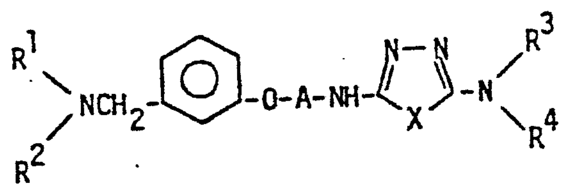

(I)

in der $R^1$ und $R^2$, die gleich oder verschieden sein können, jeweils für Wasserstoff, lineares $C_1$-$C_{10}$-Alkyl, $C_5$-$C_6$-Cycloalkyl, Amino, $C_1$-$C_3$-Alkylamino oder Di($C_1$-$C_3$-alkyl)amino stehen oder $R^1$ und $R^2$ zusammen mit dem Stickstoffatom, an das sie gebunden sind, einen gegebenenfalls ein- oder zweifach mit einer $C_1$-$C_3$-Alkylgruppe substituierten 5- bis 10-gliedrigen stickstoffhaltigen alicyclischen, heterocyclischen Ring bilden, A die Gruppierung $-(CH_2)_n-$, $-CH_2-\overset{CH_3}{CH}-CH_2-$, $-CH_2-CH{=}CH-CH_2-$ oder $-CH_2-\overset{OH}{CH}-CH_2-$ bedeutet, wobei n für 3 oder 4 steht, X ein Sauerstoff- oder Schwefelatom bedeutet, $R^3$ und

$R^4$, die gleich oder verschieden sein können, jeweils Wasserstoff, lineares oder verzweigtkettiges $C_1$-$C_6$-Alkyl, $C_5$-$C_6$-Cycloalkyl oder eine Benzyl-, Benzoyl- oder Phenylgruppe, die gegebenenfalls durch eine Nitro-, Halogen- oder $C_1$-$C_3$-Alkylgruppe substituiert ist, bedeuten oder $R^3$ und $R^4$ zusammen mit dem Stickstoffatom, an das sie gebunden sind, einen 5- bis 7gliedrigen stickstoffhaltigen alicyclischen, heterocyclischen Ring bilden, sowie ihre physiologisch annehmbaren Salze.

2. 3,4-Diazolderivate nach Anspruch 1, dadurch gekennzeichnet, daß $R^1$ für Wasserstoff oder $C_1$-$C_4$-Alkyl steht und $R^2$ für $C_5$-$C_6$-Cycloalkyl oder $C_1$-$C_4$-Alkyl steht oder $R^1$ und $R^2$ zusammen mit dem Stickstoffatom, an das sie gebunden sind, einen stickstoffhaltigen alicyclischen, heterocyclischen Ring mit 5 bis 7 Gliedern bilden.

3. 3,4-Diazolderivate nach Anspruch 1, dadurch gekennzeichnet, daß $R^1$ und $R^2$ zusammen mit dem Stickstoffatom, an das sie gebunden sind, einen Pyrrolidin-, Piperidin- oder Homopiperidinring bilden und daß A für die Gruppierung $-(CH_2)_3-$,

$-CH_2-CH(CH_3)-CH_2-$, $-CH_2-CH=CH-CH_2-$ oder $-CH_2-CH(OH)-CH_2-$ steht.

4. 3,4-Diazolderivate nach Anspruch 1, 2 oder 3, dadurch gekennzeichnet, daß $R^3$ und $R^4$ jeweils für ein Wasserstoffatom stehen.

5. Verfahren zur Herstellung von 3,4-Diazolderivaten nach den Ansprüchen 1 bis 4, dadurch gekennzeichnet, daß man

(a) zur Herstellung von Verbindungen, bei denen $R^1$, $R^2$ und A die in Anspruch 1 angegebenen Bedeutungen haben, $R^3$ für Wasserstoff steht, $R^4$ lineares oder verzweigtkettiges $C_1$-$C_6$-Alkyl, $C_5$-$C_6$-Cycloalkyl oder eine Benzyl-, Benzoyl- oder Phenylgruppe, die gegebenenfalls durch eine Nitro-, Halogen- oder $C_1$-$C_3$-Alkylgruppe substituiert ist, bedeutet und X für ein Sauerstoff- oder Schwefelatom steht, eine Verbindung der allgemeinen Formel II

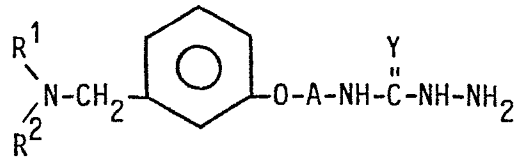

(II)

in der $R^1$, $R^2$ und A wie oben definiert sind und Y für ein Sauerstoff- oder Schwefelatom steht, mit einem Isocyaniddichlorid der allgemeinen Formel III

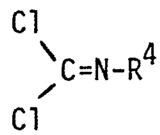

(III)

in der $R^4$ wie oben definiert ist, zu einer Verbindung der allgemeinen Formel I umsetzt oder daß man

(b) zur Herstellung von Verbindungen, bei denen $R^1$, $R^2$ und A die in Anspruch 1 angegebenen Bedeutungen haben, $R^3$ und $R^4$, die gleich oder verschieden sein können, jeweils lineares oder verzweigtkettiges $C_1$-$C_6$-Alkyl, $C_5$-$C_6$-Cycloalkyl oder eine Benzyl-, Benzoyl- oder Phenylgruppe, die gegebenenfalls durch eine Nitro-, Halogen- oder $C_1$-$C_3$-Alkylgruppe substituiert ist, bedeuten oder $R^3$ und $R^4$ zusammen mit dem Stickstoffatom, an das sie gebunden sind, einen 5- bis 7gliedrigen stickstoffhaltigen alicyclischen, heterocyclischen Ring bilden und X für ein Schwefelatom steht, eine Verbindung der allgemeinen Formel IV

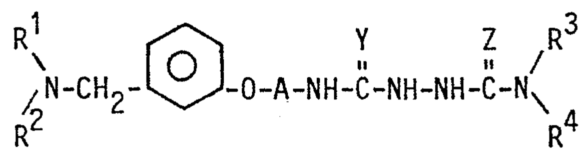

(IV)

in der $R^1$, $R^2$, $R^3$, $R^4$ und A wie oben definiert sind und Y und Z jeweils für ein Schwefelatom stehen, mit Hilfe eines Oxidationsmittels zu einer Verbindung der allgemeinen Formel I cyclisiert oder daß man

(c) zur Herstellung von Verbindungen, bei denen $R^1$, $R^2$ und A die in Anspruch 1 angegebenen Bedeutungen haben, $R^3$ und $R^4$, die gleich oder verschieden sein können, jeweils lineares oder verzweigtkettiges $C_1$-$C_6$-Alkyl, $C_5$-$C_6$-Cycloalkyl oder eine Benzyl-, Benzoyl- oder Phenylgruppe, die gegebenenfalls durch eine Nitro-, Halogen- oder $C_1$-$C_3$-Alkylgruppe substituiert ist, bedeuten oder $R^3$ und $R^4$ zusammen mit dem Stickstoffatom, an das sie gebunden sind, einen 5-bis 7gliedrigen stickstoffhaltigen alicyclischen, heterocyclischen Ring bilden und X für ein Schwefelatom steht, eine Verbindung der allgemeinen Formel IV

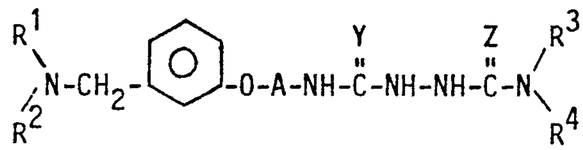

(IV)

in der $R^1$, $R^2$, $R^3$, $R^4$ und A wie oben definiert sind und entweder Y und Z gleich sind und jeweils für ein Schwefelatom stehen oder Y und Z voneinander verschieden sind und entweder für ein Sauerstoff- oder ein Schwefelatom stehen, mit Phosphoroxychlorid zu einer Verbindung der allgemeinen Formel I cyclisiert oder daß man

(d) zur Herstellung von Verbindungen, bei denen $R^1$, $R^2$ und A die in Anspruch 1 angegebenen Bedeutungen haben, $R^3$ und $R^4$, die gleich oder verschieden sein können, jeweils lineares oder verzweigtkettiges $C_1$-$C_6$-Alkyl, $C_5$-$C_6$-Cycloalkyl oder eine Benzyl-, Benzoyl- oder Phenylgruppe, die gegebenenfalls durch eine Nitro-, Halogen- oder $C_1$-$C_3$-Alkylgruppe substituiert ist, bedeuten oder $R^3$ und $R^4$ zusammen mit dem Stickstoffatom, an das sie gebunden sind, einen 5-bis 7gliedrigen stickstoffhaltigen alicyclischen, heterocyclischen Ring bilden und X für ein Sauerstoffatom steht, eine Verbindung der allgemeinen Formel IV

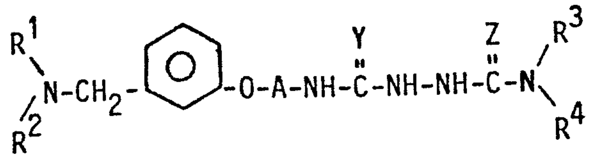

(IV)

in der $R^1$, $R^2$, $R^3$, $R^4$ und A wie oben definiert sind und Y und Z jeweils für ein Sauerstoffatom stehen, mit Phosphoroxychlorid zu einer Verbindung der allgemeinen Formel I cyclodehydratisiert oder daß man

(e) zur Herstellung von Verbindungen, bei denen $R^1$, $R^2$ und A die in Anspruch 1 angegebenen Bedeutungen haben, $R^3$ und $R^4$ jeweils Wasserstoff bedeuten und X für ein Sauerstoff- oder Schwefelatom steht, eine Verbindung der allgemeinen Formel V

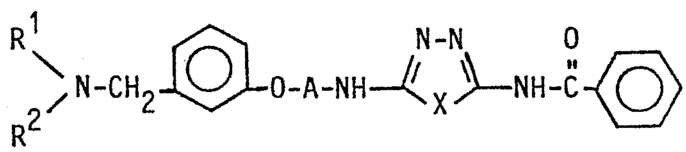

(V)

in der $R^1$, $R^2$ und A wie oben definiert sind, durch Hydrolyse zu einer Verbindung der allgemeinen Formel I umsetzt
oder daß man

(f) zur Herstellung von Verbindungen, bei denen $R^1$, $R^2$, $R^3$, $R^4$ und A die in Anspruch 1 angegebenen Bedeutungen haben und X für ein Schwefelatom steht, ein Amin der allgemeinen Formel VI

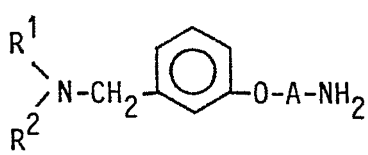

(VI)

in der $R^1$, $R^2$ und A wie oben definiert sind, mit einem 1,3,4-Thiadiazol-2-amin der allgemeinen Formel VII

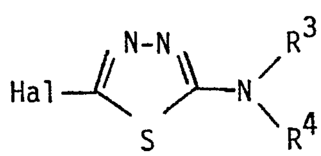

(VII)

in der Hal für ein Halogenatom steht und $R^3$ und $R^4$ wie oben definiert sind, zu einer Verbindung der allgemeinen Formel I umsetzt
oder daß man

(g) zur Herstellung von Verbindungen, bei denen $R^1$, $R^2$, $R^3$, $R^4$ und A die in Anspruch 1 angegebenen Bedeutungen haben und X für ein Schwefelatom steht, eine Verbindung der allgemeinen Formel VIII

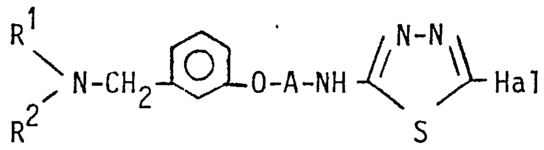

(VIII)

in der $R^1$, $R^2$ und A wie oben definiert sind und Hal für ein Halogenatom steht, mit einem Amin der allgemeinen Formel IX

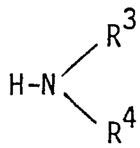

(IX)

in der $R^3$ und $R^4$ wie oben definiert sind, zu einer Verbindung der allgemeinen Formel I umsetzt

und daß man die in den Stufen (a) bis (g) erhaltene Verbindung gegebenenfalls in ihr physiologisch unbedenkliches Salz umwandelt.

6. Arzneimittel, dadurch g e k e n n z e i c h n e t , daß es eine Verbindung nach einem der Ansprüche 1 bis 4 zusammen mit einem inerten pharmazeutisch annehmbaren Träger oder Verdünnungsmittel enthält.

# PATENTANSPRÜCHE

für den Vertragsstaat AT

1. Verfahren zur Herstellung von 3,4-Diazolderivaten der allgemeinen Formel I

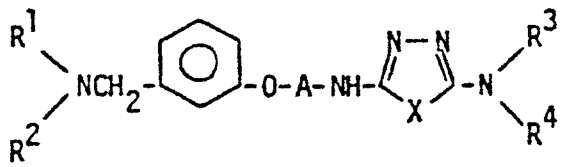

(I)

in der $R^1$ und $R^2$, die gleich oder verschieden sein können, jeweils für Wasserstoff, lineares $C_1$-$C_{10}$-Alkyl, $C_5$-$C_6$-Cycloalkyl, Amino, $C_1$-$C_3$-Alkylamino oder Di($C_1$-$C_3$-alkyl)amino stehen oder $R^1$ und $R^2$ zusammen mit dem Stickstoffatom, an das sie gebunden sind, einen gegebenenfalls ein- oder zweifach mit einer $C_1$-$C_3$-Alkylgruppe substituierten 5- bis 10-gliedrigen stickstoffhaltigen alicyclischen, heterocyclischen Ring bilden, A die Gruppierung $-(CH_2)_n-$, $-CH_2-CH(CH_3)-CH_2-$, $-CH_2-CH=CH-CH_2-$ oder $-CH_2-CH(OH)-CH_2-$ bedeutet, wobei n für 3 oder 4 steht, X ein Sauerstoff- oder Schwefelatom bedeutet, $R^3$ und $R^4$, die gleich oder verschieden sein können, jeweils Wasserstoff, lineares oder verzweigtkettiges $C_1$-$C_6$-Alkyl, $C_5$-$C_6$-Cycloalkyl oder eine Benzyl-, Benzoyl- oder Phenylgruppe, die gegebenenfalls durch eine Nitro-, Halogen- oder $C_1$-$C_3$-Alkylgruppe substituiert ist, bedeuten oder $R^3$ und $R^4$ zusammen mit dem Stickstoffatom, an das sie gebunden sind, einen 5- bis 7gliedrigen stickstoffhaltigen alicyclischen, heterocyclischen Ring bilden, sowie ihrer physiologisch annehmbaren Salze, dadurch gekennzeichnet, daß man

(a) zur Herstellung von Verbindungen, bei denen $R^1$, $R^2$ und A die oben angegebenen Bedeutungen haben, $R^3$ für Wasserstoff steht, $R^4$ lineares oder verzweigtkettiges $C_1$-$C_6$-Alkyl, $C_5$-$C_6$-Cycloalkyl oder eine Benzyl-, Benzoyl- oder Phenylgruppe, die gegebenenfalls durch eine

Nitro-, Halogen- oder $C_1$-$C_3$-Alkylgruppe substituiert ist, bedeutet und X für ein Sauerstoff- oder Schwefelatom steht, eine Verbindung der allgemeinen Formel II

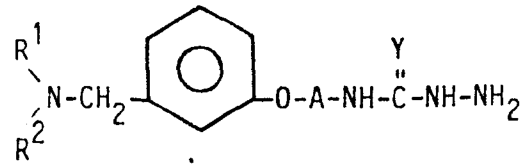
(II)

in der $R^1$, $R^2$ und A wie oben definiert sind und Y für ein Sauerstoff- oder Schwefelatom steht, mit einem Isocyaniddichlorid der allgemeinen Formel III

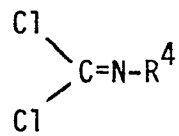
(III)

in der $R^4$ wie oben definiert ist, zu einer Verbindung der allgemeinen Formel I umsetzt oder daß man

(b) zur Herstellung von Verbindungen, bei denen $R^1$, $R^2$ und A die oben angegebenen Bedeutungen haben, $R^3$ und $R^4$, die gleich oder verschieden sein können, jeweils lineares oder verzweigtkettiges $C_1$-$C_6$-Alkyl, $C_5$-$C_6$-Cycloalkyl oder eine Benzyl-, Benzoyl- oder Phenylgruppe, die gegebenenfalls durch eine Nitro-, Halogen- oder $C_1$-$C_3$-Alkylgruppe substituiert ist, bedeuten oder $R^3$ und $R^4$ zusammen mit dem Stickstoffatom, an das sie gebunden sind, einen 5- bis 7gliedrigen stickstoffhaltigen alicyclischen, heterocyclischen Ring bilden und X für ein Schwefelatom steht, eine Verbindung der allgemeinen Formel IV

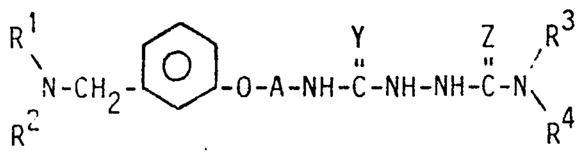
(IV)

in der $R^1$, $R^2$, $R^3$, $R^4$ und A wie oben definiert sind und Y und Z jeweils für ein Schwefelatom stehen, mit Hilfe eines Oxidationsmittels zu einer Verbindung der allgemeinen Formel I cyclisiert oder daß man

(c) zur Herstellung von Verbindungen, bei denen $R^1$, $R^2$ und A die oben angegebenen Bedeutungen haben, $R^3$ und $R^4$, die gleich oder verschieden sein können, jeweils lineares oder verzweigtkettiges $C_1$-$C_6$-Alkyl, $C_5$-$C_6$-Cycloalkyl oder eine Benzyl-, Benzoyl- oder Phenylgruppe, die gegebenenfalls durch eine Nitro-, Halogen- oder $C_1$-$C_3$-Alkylgruppe substituiert ist, bedeuten oder $R^3$ und $R^4$ zusammen mit dem Stickstoffatom, an das sie gebunden sind, einen 5- bis 7gliedrigen stickstoffhaltigen alicyclischen, heterocyclischen Ring bilden und X für ein Schwefelatom steht, eine Verbindung der allgemeinen Formel IV

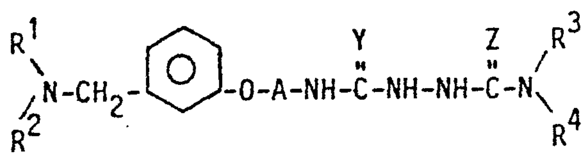

(IV)

in der $R^1$, $R^2$, $R^3$, $R^4$ und A wie oben definiert sind und entweder Y und Z gleich sind und jeweils für ein Schwefelatom stehen oder Y und Z voneinander verschieden sind und entweder für ein Sauerstoff- oder ein Schwefelatom stehen, mit Phosphoroxychlorid zu einer Verbindung der allgemeinen Formel I cyclisiert oder daß man

(d) zur Herstellung von Verbindungen, bei denen $R^1$, $R^2$ und A die oben angegebenen Bedeutungen haben, $R^3$ und $R^4$, die gleich oder verschieden sein können, jeweils lineares oder verzweigtkettiges $C_1$-$C_6$-Alkyl, $C_5$-$C_6$-Cycloalkyl oder eine Benzyl-, Benzoyl- oder Phenylgruppe, die gegebenenfalls durch eine Nitro-, Halogen- oder $C_1$-$C_3$-Alkylgruppe substituiert ist, bedeuten oder $R^3$ und $R^4$ zusammen mit dem Stickstoffatom, an das sie gebunden sind, einen 5- bis 7gliedrigen stickstoffhaltigen alicyclischen, heterocyclischen Ring bilden und X für ein Sauerstoffatom steht, eine Verbindung der allgemeinen Formel IV

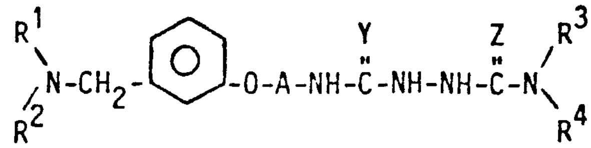 (IV)

in der $R^1$, $R^2$, $R^3$, $R^4$ und A wie oben definiert sind und Y und Z jeweils für ein Sauerstoffatom stehen, mit Phosphoroxychlorid zu einer Verbindung der allgemeinen Formel I cyclodehydratisiert oder daß man

(e) zur Herstellung von Verbindungen, bei denen $R^1$, $R^2$ und A die oben angegebenen Bedeutungen haben, $R^3$ und $R^4$ jeweils Wasserstoff bedeuten und X für ein Sauerstoff- oder Schwefelatom steht, eine Verbindung der allgemeinen Formel V

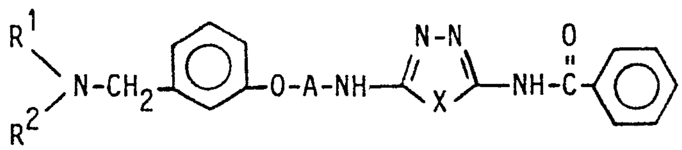 (V)

in der $R^1$, $R^2$ und A wie oben definiert sind, durch Hydrolyse zu einer Verbindung der allgemeinen Formel I umsetzt oder daß man

(f) zur Herstellung von Verbindungen, bei denen $R^1$, $R^2$, $R^3$, $R^4$ und A die oben angegebenen Bedeutungen haben und X für ein Schwefelatom steht, ein Amin der allgemeinen Formel VI

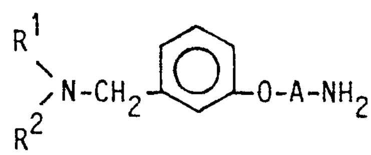 (VI)

in der $R^1$, $R^2$ und A wie oben definiert sind, mit einem 1,3,4-Thiadiazol-2-amin der allgemeinen Formel VII

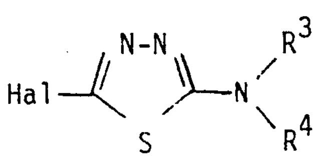 (VII)

in der Hal für ein Halogenatom steht und $R^3$ und $R^4$ wie oben definiert sind, zu einer Verbindung der allgemeinen Formel I umsetzt oder daß man

(g) zur Herstellung von Verbindungen, bei denen $R^1$, $R^2$, $R^3$, $R^4$ und A die oben angegebenen Bedeutungen haben und X für ein Schwefelatom steht, eine Verbindung der allgemeinen Formel VIII

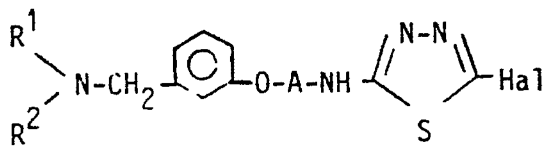

(VIII)

in der $R^1$, $R^2$ und A wie oben definiert sind und Hal für ein Halogenatom steht, mit einem Amin der allgemeinen Formel IX

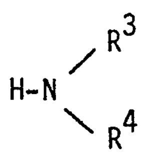

(IX)

in der $R^3$ und $R^4$ wie oben definiert sind, zu einer Verbindung der allgemeinen Formel I umsetzt

und daß man die in den Stufen (a) bis (g) erhaltene Verbindung gegebenenfalls in ihr physiologisch unbedenkliches Salz umwandelt.

2. Verfahren nach Anspruch 1 zur Herstellung von Verbindungen, bei denen $R^1$ für Wasserstoff oder $C_1$-$C_4$-Alkyl steht und $R^2$ für $C_5$-$C_6$-Cycloalkyl oder $C_1$-$C_4$-Alkyl steht oder $R^1$ und $R^2$ zusammen mit dem Stickstoffatom, an das sie gebunden sind, einen stickstoffhaltigen alicyclischen, heterocyclischen Ring mit 5 bis 7 Gliedern bilden.

3. Verfahren nach Anspruch 1 zur Herstellung von Verbindungen, bei denen $R^1$ und $R^2$ zusammen mit dem Stickstoffatom, an das sie gebunden sind, einen Pyrrolidin-, Piperidin- oder Homopiperidinring bilden und

A für die Gruppierung $-(CH_2)_3-$,

$$-CH_2-\overset{\displaystyle CH_3}{\overset{|}{C}H}-CH_2-, \quad -CH_2-CH{=}CH-CH_2- \text{ oder } -CH_2-\overset{\displaystyle OH}{\overset{|}{C}H}-CH_2- \text{ steht.}$$

4. Verfahren nach Anspruch 1, 2 oder 3 zur Herstellung von Verbindungen, bei denen $R^3$ und $R^4$ jeweils für ein Wasserstoffatom stehen.